# EUROPEAN PATENT SPECIFICATION

(11) **EP 3 193 730 B1**
(45) Date of publication and mention of the grant of the patent: **27.05.2020**
(21) Application number: 15766571.2
(22) Date of filing: 11.09.2015
(51) Int. Cl.: A61B 10/00, A61B 5/07

(54) **SAMPLING DEVICE**
PROBENAHMEVORRICHTUNG
DISPOSITIF D'ÉCHANTILLONNAGE

(30) Priority: 17.09.2014 GB 201416457; 17.09.2014 GB 201416453
(43) Date of publication of application: 26.07.2017
(73) Proprietor: Mars, Incorporated, McLean, VA 22101 (US)
(72) Inventor: WRIGGLESWORTH, David, Waltham-on-the-Wolds Leicestershire LE14 4RT (GB); BRADLEY, William James, Cambridge Cambridgeshire CB1 3TB (GB)
(74) Representative: Carpmaels & Ransford LLP
(86) International application number: PCT/GB2015/052648
(87) International publication number: WO 2016/042302

(56) References cited:
- WO-A1-2013/120184
- US-A- 4 481 952
- US-A- 5 395 366

## Description

The present invention relates to a sampling device. The sampling device comprises a separate compartment (30) that is ejected creating an increased space or volume for a sample to be stored The sampling device of the present invention is suitable for collecting a sample in an aquaculture environment, enclosed system or in the gastrointestinal tract of a human or an animal. The invention also relates to a method of orally administering the device to an animal and recovering the device to carry out analysis on the collected sample for diagnosing the health of the gastrointestinal tract and determining nutrient absorption and digestibility.

US patent 5,395,366 (D Andrea David T et al) describes an ingestible capsule and process for sampling fluids in the alimentary canal. The capsule signals a remote receiver as it progresses through the alimentary tract in a previously mapped route and upon reaching a specific site is remotely triggered to capture a fluid sample in the alimentary canal. The capsule comprises an electric power source, a radio signal transmitting means, a remote actuatable activating means, fluid sample storage means and a sampling means, encased in an essentially non-digestible outer shell.

International patent application WO 2013/120184A1 (Micropharma Ltd) describes an ingestible medical device with a rotatable element. The application describes a device which is capable of performing a collection of samples and distributing substances within the GI tract of a body. The device comprises a chamber enclosure with an access port, wherein one of the chamber enclosures and the storage sub-unit are rotatable to allow for aligning the access port with a chamber opening.

US patent 4,481,952 (Pawelec Jerzy) describes a capsule for obtaining samples from the gastric and/or intestinal fluids, the capsule defining an inner fluid receiving chamber having an inlet port which is normally sealed off from the exterior of the capsule by way of a blocking means, including a mass the properties of which change upon contact with the suitable fluids present in the digestion tract. This contact causes the means to communicate with the inlet port with the outside of the capsule allowing a sample of the fluid to be aspirated into the chamber.

The ability to directly sample substances within the gastrointestinal tract of an animal is a key enabling technology for diagnosing the health of the gastrointestinal tract; understanding ileal digestion, fermentation in the colon and for investigating the relationship between specific dietary components and the biological, chemical and physio-chemical properties in the gastrointestinal tract of humans and/or animals. This includes nutrient absorption, drug metabolism, microorganism distribution, immunological status and so on.

Analysing a sample of substance, such as a gas, a solid particle or a liquid from the gastrointestinal tract of an animal, can provide information on the pH, enzyme activity, microbial load/content and molecular composition of the sampled material including nutrient load and degree of processing information enabling the assessment of health status and diagnosis of diseases, and in particular diseases of the gastrointestinal tract can also be obtained through determination of immune factors or general immunological status, characterisation of the microflora present and presence/absence of microbial pathogens or microbes associated with health.

Early detection, identification of and location of abnormal conditions can be critical for definitive diagnosis and/or treating various pathologies. Particular control over the site of digesta sampling is also vital to the understanding of health and disease since conditions are generally site specific for example relating to either the stomach, ileum or colon as opposed to affecting the entire gastrointestinal tract. Site specific sample acquisition is also of use for understanding dietary impact and nutrition, for example, ilea processing is an important factor in diet design since the efficiency of digestion and absorption within the small intestine impacts on the luminal contents present in the distal portion of the gastrointestinal tract. Fermentation by the intestinal flora within the colon is dependent on the luminal contents reaching the colon and hence factors such as incomplete protein digestion have been associated with the formation of toxic compounds, including ammonia, dihydrogen sulphide, in-doles and phenols, which in turn have been shown to increase the risk of colon cancer in humans. In the design of diets for "production animals", it is also important to enhance the efficiency of nutrient availability in foodstuffs and the environmental impact of waste products from the animal.

Whilst total apparent digestibility (as measured by the percentage of ingested nutrient that is recovered in the faeces) is a key measure, this methodology has a number of shortcomings, due to the contamination of faeces with metabolic waste products and substances of non-dietary origin. Ileal digestibility is therefore a more appropriate measure of nutrient delivery. Furthermore, the outflow of the terminal ileum into the caecum can be considered as the substrate upon which the hind gut microflora act. The colonic digesta or luminal contents may therefore provide a predictor or allow assessment of the nutrients available for fermentation by the resident gastrointestinal microflora. Consequently the ability to measure a broad range of chemical (e.g. protein, carbohydrates, fats, non-starch polysaccharides, micronutrients, anti-nutritional factors), biological and physio-chemical (e.g. viscosity) properties from within the ileal and colonic digesta represents a significant development that may in turn enable a step-change in our understanding of the effects of specific dietary components on digestive processes and its products. Such digestion products may include faecal characteristics such as faeces quality and consistency and intestinal gas or flatulence. The development of these enabling technologies may also, enable diagnosis and research into gastrointestinal diseases.

There is an ongoing need to improve known sampling devices, such that a larger volume of sample may be acquired, and sampling of liquids ranging in viscosity as well as solid samples can be acquired. These improvements to the sampling device allow a greater range of analytical tests to be carried out on the sample and enhance the range of samples for acquisition. Furthermore, the ability to alter the point within the gastrointestinal tract at which the sample is captured allows investigation of the changes in factors involved in digestion (pH, enzymes emulsifying agents etc.), dietary processing; nutrient absorption; microbial populations; immune factors and digesta viscosity throughout the length of the gastrointestinal lumen. Sampling may therefore proceed within the stomach, ileum, colon and caecum allowing sampling site to be adjusted based on the nature of the sample required.

The device of the present invention is simple, inexpensive, reliable and easy to use, without the need for constant monitoring and may be reusable. The device of the present invention can be recovered and the sample easily extracted and analysed using a variety of biological, chemical and physical assays. Both liquid and solid substances can be sampled by the device of the present invention.

The device of the invention is able to log a change in the environment over a long period of time, e.g. for 24 hours. In particular, the change in the environment along the gastrointestinal tract of an animal. The device of the invention is able to download the data stored to a computer and be easily reprogrammed to be reused. The device of the invention is able to detect and log the time pH and/or temperature at which the sample is obtained.

In particular, the device of the invention is able to collect a large amount of sample as a result of its separate compartment being ejected creating an increased volume or space for a sample to be collected and stored.

In a first aspect of the invention there is provided a sampling device. The sampling device comprises a housing (20), wherein the housing comprises a chamber, at least an opening (44), an actuation means (50) and a separate compartment (30), wherein the separate compartment is releasably retained within the housing, the actuation means enables an internal substance to be drawn in through the opening into the chamber and simultaneously pushes the separate compartment along the housing until the separate compartment is ejected from the housing of the sampling device, thereby creating an increased space or volume for receiving and storing a sample within the chamber of the sampling device.

In some embodiments, the sampling device can be used for sampling any form of closed system, such as fish tanks, processing tanks, bioprocessing, various agricultural systems or any system where human/physical intervention is beneficial and/or industrial or factory pipes.

In particular embodiments, the sampling device of the invention is for sampling internal substance from within the gastrointestinal tract of an animal.

The terms "sample", "substance" and "internal substance" are used interchangeably and refers to any liquid, solid, particle or gas, which can be sampled by the device, more specifically these terms may also be referred to as digesta or luminal contents or digestion products when referring to sampling within the gastrointestinal tract of an animal. Liquid can for example be found in the stomach, small and large intestine. Such liquids may contain solids or species in solution (or suspensions) such as dietary components, drugs, food components; digestion products, microbial metabolites, gases, such as oxygen, hydrogen, carbon dioxide, methane, hydrogen sulfide, etc. can also be found.

In particular, the device is designed to withstand any pressure (such as chewing) and/or change in environment. In particular, the device is designed to travel along the gastrointestinal tract of an animal. The device must be able to withstand peristalsis of the gastrointestinal tract, as well as the chemical and mechanical environment of the gastrointestinal tract.

Different materials may be used for each different component of the sampling device. In particular, the material used determines the texture and/or hardness of the device. The materials can be hard, soft, smooth and/or malleable and the preference of the material used is dependent on its use.

Typically, the device can be made of any non-digestible, non-biodegradable, non-immunogenic, non-bioreactive or impermeable material. In particular, the material used to make the device can be any biologically inert polymeric materials, such as acrylonitrile butadiene styrene (ABS) polytetrafluoroethylene (PTFE), polyethylene, polyvinyl chloride, acrylics and the like, ceramics or metals, for example stainless steel, preferably smooth surfaced for ease of ingestion and transit and with at least one radio opaque surface or inclusion such that it can be observed radiographically if required.

Thermoplastic materials can be used, such as polycarbonates, acrylonitrile butadiene styrene (ABS), High-density polyethylene (HDPE), Low-density polyethylene (LDPE), Polyether ether ketone (PEEK) or Polypropylene (PP).

Preferably, the external surface of the device is made of polycarbonate. The polycarbonate may be translucent to aid in the visual assessment of the contained sample. Other components are preferably made from polytetrafluoroethylene (PTFE). In particular, PTFE may be used for internal components of the device where the flexible nature of this material allows a seal to be formed between two adjacent surfaces.

All materials used in the sampling device are inert and safe for food and/or medical use.

In some embodiments, the device can be in a shape of a capsule or a pill. The capsule can be cylindrical with rounded, conical or flattened ends. The device can be partially spherical in shape.

The sampling device, and in particular the different components of the sampling device, are fabricated with conventional tools and/ or methods known in the art. In particular, the sampling device and components may be fabricated using additive techniques such as 3D printing or by reductive techniques such as CNC machining known to the art.

In particular, the device is preferably suitable for swallowing and for an animal to ingest. Capsules known in the art have dimensions of 26mm to 30mm x 11 mm to 15mm (length x width).

The particular advantage of the present invention is that although the sampling device may have similar dimensions as those known in the art, a separate compartment (30) within the sampling device is ejected creating an increased volume or space for a sample to be received and/or stored. Depending on the animal, size, breed and/or species, the device will vary in size as described herein. The dimensions of the device can range from about 10mm to 70mm in length to 3mm to 25mm in width. In particular, the dimensions of the device may be about 10-15mm and about 3- 7mm, about 1 5-25mm x about 7-12mm, about 20-30mm x about 10-17mm, about 30-40mm x about 15-20mm, about 10-20mm x about 3-20mm, about 35-65mm x about 7-23mm, or about 40- 70mm x about 10-25mm and/or any combination thereof.

Depending on the size of the capsule used, the device of the invention is capable of obtaining a sample volume from about 0.11 ml to about 20ml. In particular, the sample volume can be about 0.11 ml to 3ml, about 1 ml to 5ml, about 3ml to 7ml, about 7ml to 13ml, about 10ml to 15ml, about 13 ml to 17ml or about 15ml to 20ml and/or any combinations thereof. In a particular example of the invention, the device may have an internal volume of 1 .53cm<3>and may be capable of obtaining and storing a sample volume of 1 .3ml, which is 85% of the available internal volume.

The device can most preferably have dimensions of about 20 to 25mm x about 9 to 12mm (length x width).

The device is of a modular design in that it comprises one or more components. In particular, the device is assembled from one or more components. One component may comprise an actuating means (50) and an opening (44). Another component may comprise a housing (20). Another component may comprise a stand-alone separate compartment (30).

The different components of the sampling device may be formed separately and easily assembled together to form the sampling device and/or separate parts of the sampling device.

In some embodiments, the sampling device can comprise two halves connected at least to one another by one or more elements and/or the housing (20).

In some aspects of the invention the sampling device comprises a housing (20), wherein the housing comprises a chamber, at least an opening (44), an actuation means (50) and a separate compartment (30), wherein the separate compartment is releasably retained within the housing by a retention means (100) and wherein the actuation means enables an internal substance to be drawn in through the opening into the chamber and simultaneously pushes the separate compartment along the housing until said separate compartment is ejected from the housing of the sampling device, thereby creating an increased space or volume for receiving and storing a sample within the chamber of the sampling device.

In yet other aspects of the invention, the sampling device comprises a housing (20), wherein the housing comprises a chamber, at least an opening (44), an actuation means (50) and a separate compartment (30), wherein the separate compartment is releasably retained within the housing by a retention means (100) that is activated by a trigger means (70), wherein the actuation means enables an internal substance to be drawn in through the opening into the chamber and simultaneously pushes the separate compartment along the housing until said separate compartment is ejected from the housing of the sampling device, thereby creating an increased space or volume for receiving and storing a sample within the chamber of the sampling device.

In another aspect of the invention, there is provided a sampling device comprising a housing (20), wherein the housing comprises a chamber, at least an opening (44), an actuation means (50) and a separate compartment (30), wherein the separate compartment is releasably retained within the housing by a retention means (100) that is a material that reacts to changes in the external environment of the device and wherein the actuation means enables an internal substance to be drawn in through said opening into said chamber and simultaneously pushes the separate compartment along housing until said separate compartment is ejected from the housing or the sampling device, thereby creating an increased space or volume for receiving and storing said sample within the chamber of the sampling device.

In another aspect of the invention, there is provided a sampling device housing (20), wherein the housing comprises a chamber, at least an opening (44), an actuation means (50) and a separate compartment (30), wherein the separate compartment is releasably retained within the housing by a retention means, wherein the retention means is an interlocking mechanism between the housing and the separate compartment and wherein the actuation means enables an internal substance to be drawn in through the opening into the chamber and simultaneously pushes the separate compartment along the housing until the separate compartment is ejected from the housing of the sampling device, thereby creating an increased space or volume for receiving and storing a sample within the chamber of the sampling device.

In yet another aspect of the invention, there is provided a sampling device comprising a housing (20), wherein the housing comprises a chamber, at least an opening (44), an actuation means (50) and a separate compartment (30), wherein the separate compartment is releasably retained within the housing by a retention means and wherein the actuation means enables an internal substance to be drawn in through the opening into the chamber and simultaneously pushes the separate compartment along the housing until the separate compartment is ejected from the housing of the sampling device, thereby creating an increased space or volume for receiving and storing a sample within the chamber of the sampling device, wherein the actuation means is tethered to a closure means (80) and wherein the movement of the actuation means results in the closure means blocking the opening.

The sampling device comprises a housing (20). The housing comprises a chamber, at least an opening (44) and a separate compartment (30). In particular, the housing is an enclosure, which holds the actuating means (50) and the separate compartment within the sampling device.

In some embodiments, the housing can be made from polycarbonate.

In particular, the sampling device of the invention is re-usable. In particular, the separate component (30) can be unscrewed and reused multiple times. The entire device can be re-used. In certain embodiments of the invention, the material that reacts with the external environment of the device, the battery(s) (62), and/or the trigger means (70) may require replacing, e.g. Eudragit washer, wax washer, fuse, or the battery.

The housing (20) of the sampling device of the invention may have one or more openings.

In particular, the sampling device has at least one opening (44). The opening may be an inlet and/or an outlet. The opening may be an aperture or a hole at one or either end of the housing (20). In particular embodiments, the opening is an inlet, which allows sample into the housing of the sampling device, preferably into the chamber. Alternatively, the opening can be an outlet which allows the separate compartment (30) to be ejected from the housing of the sampling device. In particular, the housing may have two openings, one on each end of the housing on opposing ends of the sampling device (an inlet and an outlet).

In some embodiments, the inlet opening can be a valve. The valve can be any valve known in the art and will depend on the use of the sampling device. The valve includes an aperture. The valve can be made from rubber or synthetic elastomer. Preferably, the valve is made of elastomer which is resistant to low pressure therefore opening the aperture. In other embodiments, the opening can be an inlet and an outlet. The opening can be a two way valve. Most preferably, the opening is a one-way valve.

In some embodiments, the opening includes an aperture without the valve.

The sampling device comprises an actuation means (50).

In particular, the actuation means is arranged in a first configuration and capable of moving to a second configuration within the housing (20) of the sampling device. The first configuration is a compressed state and the second configuration is an expanded state. The actuation means assists in drawing a sample into the housing of the sampling device, preferably into the chamber.

In particular, the actuation means enables a sample to be drawn in through the opening into the chamber of the housing (20) of the device and simultaneously pushes the separate compartment (30) along the housing until the separate compartment is ejected from the device.

An actuation means (50) can be any object or element that is capable of storing internal energy for a period of time within the device. In particular, an actuation means can be a system and/or an object capable of putting another object or element into motion and/or action, for example providing the force to move the separate compartment (30) from a retained and compressed state to an uncompressed and ejected state.

The actuation means can include a spring (52), a chemical reaction (releasing gas and producing pressure), an electronically powered actuator (such as a motor or a pump), compressed air or a vacuum energy store.

The actuation means retains an amount of internal energy, preferably for a pre-determined time in various pre-determined conditions.

The actuation means is held in compression when the separate compartment is in place.

An actuation means may comprise a resilient member. In particular, it may comprise any object that is capable of storing kinetic energy and/or capable of movement. A resilient member may be a spring and/or a plunger/piston.

The actuation means at least comprises a plunger/piston (54). The actuation means can comprise a plunger/piston and a spring, a plunger/piston and an electronically powered actuator, etc. In some embodiments, the plunger/piston may be moved from the first configuration to the second configuration within the housing of the sampling device by means of a chemical reaction within the housing of the device which releases gas and/or produces pressure.

The movement of the actuation means (e.g. the spring that is coupled to the plunger/piston or the plunger/piston forced along the housing by other means) pushes the separate compartment (30) simultaneously out of the housing (20) of the sampling device.

In particular, the actuation means (50) draws up a sample through the opening (44) of the sampling device into the chamber and simultaneously pushes the separate compartment (30) along the length of the housing of the sampling device until the separate compartment is ejected from the housing of the sampling device.

In particular embodiments, the actuation means (50) includes a spring (52).

Typically a spring (52) is an elastic object, such as a coil of wire, which regains its original shape after being compressed or extended and releasing stored energy during the return to the original state. A spring can include any type of spring such as a coil spring, conical spring, a torsion spring, a compression spring, or a wave spring, etc. The spring can be made of any material that is resistant and elastic, such as stainless steel or other metal or alloy.

In a particular embodiment, the spring (52) acts as an actuator. The device is held in a compressed state by the separate compartment (30). The spring can drive the plunger/piston along the housing (e.g. along the length of the housing) pushing the separate compartment out of the housing of the sampling device, converting the actuation means (50) from its first compressed configuration to the second expanded configuration. The driving force of the spring creates a short time framed vacuum, as it expands along the housing and/or a capillary action in which the sample is drawn into the housing of the sampling, preferably into the chamber through the opening.

When the separate compartment (30) is not in place (i.e. has been ejected from the housing of the sampling device), a stopper means at the end of the housing of the sampling device prevents the plunger/piston from exiting the device.

The motion of the separate compartment being ejected from the housing of the sampling device is caused by the actuation means.

Preferably, the actuation means (50) is a spring (52) and a plunger (54) arrangement or a spring and a piston arrangement.

In some embodiments, the actuation means (50) is coupled at one end of the housing of the sampling device and the separate compartment is at least releasably retained by a retention means (100) within the housing at the opposing end of the sampling device.

In particular, when the actuation means reaches the opposite end of the housing (20) it is capable of forming a seal, so that the device is sealed.

In particular, the actuation means is coupled to the valve. In particular, the actuation means and the valve are coupled in a holder (40). The holder can be made from any material, in particular from polycarbonate. When coupling the actuation means and the valve into the holder, a retaining ring (100) may be used, such as an O-ring (100B).

The term "coupled" refers to two or more objects which are attached to one another directly or through one or more intermediate elements, or are held adjacent to one another.

In particular, the actuation means (50) and the opening (44) are an individual component of the sampling device, which is capable of attaching and/or fixing to the housing (30) of the sampling device. Preferably, the actuation means and the opening are coupled together into an individual compartment which screws into one end of the housing of the device.

The plunger/piston may be made from poly tetrafluoroethylene.

In particular, the actuation means (50) (e.g. plunger/piston) is adapted with a protrusion that is capable of engaging with the separate compartment (30). The protrusion is positioned centrally to the separate compartment, such that the separate compartment travels in a linear manner when being ejected under the force exerted by the actuation means. In particular, the protrusion is on the upper surface of the actuation means. The protrusion may include a magnet, an inductive coil or a light source.

In particular embodiments of the invention, the sampling device includes a closure means (80).

A closure means (80) is an object that obstructs an aperture. In particular, the closure means can block any one of the openings. The closure means, can be any form of element or object that is capable of blocking or plugging the opening of the device, such as a ball of thermoplastic material, or similar material thereby forming a seal in the opening of the device. The closure means assists in closing the opening. The closure means can be a block, ball, lid or other. In particular, the closure means can be tethered to the actuation means. In particular, the closure means can be tethered to the actuation means and the retention means.

The term "tether", "tethering" or "tethered" refers to any form of a cord, fixture, or flexible attachment that anchors something movable to another element or a part which can also be moveable or fixed.

In another embodiment, the actuation means (50) (e.g. the plunger/piston) is tethered to a closure means (80), which is pulled into tension as the actuation means moves along the housing of the sampling device. In particular, as the actuation means is moved, the tether (82) is tensed and driven into the housing of the sampling device, until the closure means reaches the opening. The closure means can be pulled or pushed into the opening (44), forming a closed device. Simultaneously, the actuation means has drawn sample into the chamber of the device and the separate component (30) is ejected. Thereby, the sample is sealed within the device.

In particular, the housing of the sampling device includes a stopper means.

The stopper means prevents the actuation means (50) (e.g. plunger/piston) from ejecting from the housing (20). The stopper means can be any protrusion and/or additional element or object that obstructs or halts another object at a given point. In particular the stopper means is a protrusion that stops the actuation means from exiting the housing. The stopper means can be located at the end of the housing in which the separate compartment (30) is ejected. It functions by stopping the actuation means (e.g. plunger/piston) from being released further and along with the separate compartment that is ejected from the device. In particular, the stopper means acts to stop the actuation means from being ejected and thereby seals the device.

The stopper means can be a lip, lug, protrusion, or pin.

The sampling device comprises a separate compartment (30), wherein the separate compartment is a stand-alone compartment. The separate compartment can be inserted loosely and/or fitted into the housing of the sampling device.

In particular, the separate compartment (30) is an enclosed compartment, which can be unscrewed at any given time for reusing. The separate compartment is composed of two separate components, a housing (32) and a lid (34). The lid is coupled with the associated electronics and a power source (e.g. a battery). The lid can screw into the housing of the separate compartment and be tightly sealed. In particular, the lid may further comprise an O-ring (36).

In particular, the separate compartment is air and water tight.

The interface of the lid (34) and housing (32) of the separate compartment (30) may be coated with a food safe silicon grease to aid sealing. The associated electronic components may be encased in Polytetrafluoroethylene (PTFE) tape to prevent contamination with liquid, grease, or other material that may interfere with the correct functioning of the electronic components.

In some embodiments, the device and/or the separate component (30) can be reusable. In particular, the separate compartment can be reprogrammed. For example, the device can be reconfigured e.g. to a different sampling rate. The device can be user defined, rather than fixed. The device allows some flexibility in its configuration. This is achieved through the serial communications clip and associated software.

In some embodiments, the separate compartment includes any associated electronics circuits and at least a battery (62). Preferably, the separate compartment includes at least a battery, a sensor and a microprocessor.

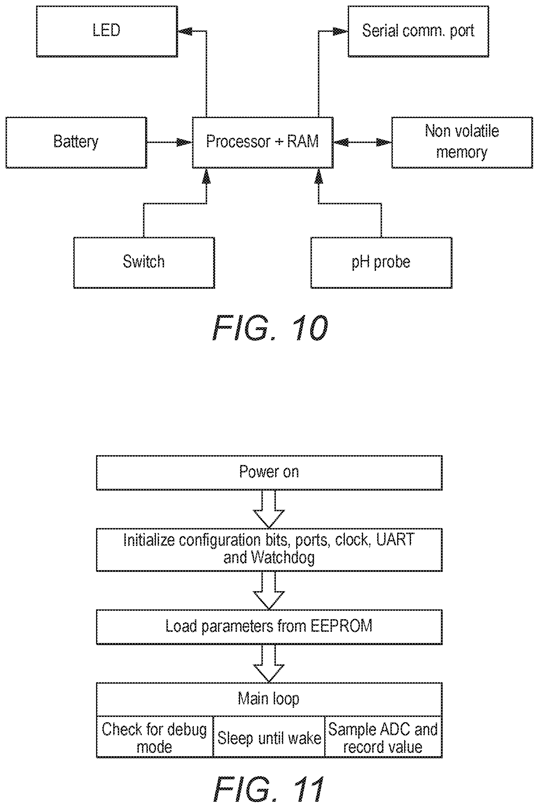

Typically, the device may comprise a printed circuit board (PCB) (66) that includes a microcontroller (PIC microcontroller), a sensor (such as a glass pH microelectrode and a pH reference microelectrode (69)), a light emitting diode, a release detect switch (60), a battery connection, various resistors and capacitors and/or a power source. Figure 10 is a schematic diagram to show the different electrical components that can be in the separate compartment of the device.

The sampling device may include a battery (62). In particular, such as battery is within the separate compartment (30).

The battery (62) can be any form of battery known in the art, such as a pin of carbon monofluoride lithium or a coin cell. Coin cell batteries can be made of alkaline, lithium, silver, etc. and or combinations thereof and are available in different sizes.

In particular, the battery is capable of running for at least 24 hours. The battery may be rechargeable.

The separate compartment may comprise one or more batteries (62).

Essentially the device can be turned on when the battery is inserted; the device initializes and then repeatedly goes through the following main loop cycle, see Figure 11.

Typically, on being powered on the software initialises and defines configuration bits, ports, clock, universal asynchronous receiver/transmitter and watchdog timer.

Parameters are loaded from the electronically erasable programmable read-only memory, wherein the parameters to be loaded can be a device ID, a log address, sample rate, pre-wake time, burst count and sample number.

The device ID is the serial number of the device. The log address is the address at which the next logged data will be stored. The sample rate is the sample rate of the data logging. The pre-wake time is set to allow the microprocessor to reach normal operating condition. The burst count is the number of samples taken per interval and sample number is the number of samples taken before the sample is obtained and recorded.

The device then enters a main loop, wherein it checks for the presence of a serial interface. If the serial interface is present, the device enters a debug mode in which various parameters can be manipulated. In the absence of a serial interface, the watchdog timer instigates a wake event during which data is sampled from the analogue digital converter and recorded to flash memory. The device can then reenter sleep mode until the watchdog timer instigates a further wake event.

In some embodiments, the separate compartment (30) further comprises flash memory, which is capable of recording data. The data is stored and then downloaded when the separate compartment is recovered or is transmitted live to a remote facility (i.e. via wireless data transmission).

The wireless data transmission system may consist of two parts. The first part is the device, which contains radio frequency transmitter circuitry, antenna and processing means with firmware to control transmissions, for example a microchip inserted in the device (such as radio frequency identification (RFID)). The second part consists of radio frequency receiver circuitry and an antenna. Such wireless transmission is also effective in locating the device.

The second part has a range of possible embodiments, including but not limited to a mat which can be placed on the floor of a kennel; a small unit which can be the floor, wall or ceiling mounted; a collar, belt, jacket, boot, ear piercing or another form which can be carried externally by the animal or placed in the external environment of the closed system in which the device is being used (such as outside a fish tank, outside factory pipes, etc).

The second part also includes a means of storing received data and communicating that data to operators or other equipment. Communication to the user either via the first part or second part of the wireless data transmission may be in the form of illuminated indicators, speakers or sounders, or a graphical display, for example available signals (such as buzzers).

Communicated data between the first part and the second part may include but is not limited to measurements of pH by the device; measurements of temperature by the device; measurements from other sensors by the device, other status information from the device, such as battery level, sample capture status etc; data that has been intelligently processed by the device and is derived from other readings, for example alerts to indicate the estimated position of the device within the gastrointestinal tract (i.e. leaving the stomach; entering ileum, etc.).

Communication by the second part to other equipment may include standard or proprietary wired communications interfaces (e.g. Ethernet, USB, serial cable etc.), standard or proprietary wireless communications (e.g. Bluetooth, GSM, wifi), removable data storage such as SD card, USB data 'key' etc.

The separate compartment may comprise a pressure switch. In particular, the switch is adapted to record the timing at which the separate compartment is ejected from the housing of the sampling device.

The switch can be a contact switch or a non-contact switch. The contact switch may be a reed switch, pressure switch or a non-contact switch which can be an inductive coil, built within the microprocessor or an optical switch, such as an LED and photodiode pair.

Preferably, the switch (60) can be a pressure switch.

The pressure switch interfaces with a small protrusion on the upper surface of the actuation means (50) (e.g. the plunger/piston). The actuation means and the separate compartment (30) apply pressure to one another and thereby maintain a contact to one another by means of the protrusion and pressure switch. In particular, when the device is actuated by the retention means, the separate compartment is at least partially released and at least partly disengaged from the actuating means.

In particular, the switch (60) is closed when the separate compartment (30) and the actuation means (50) are engaged. When the separate compartment and the actuation means are disengaged the switch is open. Typically, when the switch is open, it results in the timing at which the sample has been obtained and the separate compartment has been ejected. This is usually at the point in which the separate compartment is ejected from the device.

In alternative embodiments, the switch can be a reed switch. The reed switch can be kept in the closed configuration by a magnet located on the actuation means in particular within the protrusion on the actuation means. When the separate compartment is ejected, the magnet and reed switch are no longer in contact, the reed switch is therefore open and provides a signal that a sample has been captured.

In other embodiments, the switch can be an inductive coil. The inductive coil arrangement exists between the actuation means and the separate compartment, whereby a current is induced in the coil of the separate compartment. When the separate compartment is ejected, the induced current in the coil of the separate compartment is no longer present, signalling that the sample has been captured.

In yet other embodiments, the switch can be an optical switch. The optical switch can be a small light source (e.g. a light emitting diode) and a light dependent resistor that are arranged between the actuation means and the separate compartment, such that the light affects the electrical resistance of the light dependent resistor. When the separate compartment is ejected, the light source is no longer able to affect the light dependent resistor and the resulting change in resistance is interpreted as a signal that the sample has been captured.

The sampling device may include a sensor. In particular, the separate compartment includes the sensor. In particular, the sensor may be a pH sensor (69) and/or a temperature sensor. Other sensors that are capable of sensing and signaling other environmental factors, such as pressure, solute etc can also be used in the device.

In particular, the sensor may protrude from the surface of separate compartment so that it is exposed to the external environment of the sampling device.

In preferred embodiments, the sensor is a pH sensor/meter, which can include a glass pH microelectrode and a pH reference microelectrode (68).

A typical pH meter consists of a measuring probe (such as a glass electrode) and a reference probe connected to an electronic meter that measures and displays or logs the pH reading. A variety of pH meters are known in the art that can be used in this invention. The preferred pH sensing components in the present invention consist of a glass microelectrode coupled with a reference microelectrode. Alternative embodiments may consist of ion selective field effect transistors, solid state reference electrodes, or other suitable technology readily known in the art.

In particular, the printed circuit (66) of the device may comprises a pH circuit. The circuit can log the pH level during the transit of the device (e.g. along the gastrointestinal tract), wherein the circuit comprises of at least a pH electrode, a reference electrode, a microprocessor (64), a switch assembly and a power source.

Typically, the software and electronics will interface with a miniaturised pH probe input to an analogue digital converter, a reference voltage to the pH reference electrode, a switch contact that detects the device configuration status as either open or closed, a universal asynchronous receiver/transmitter (UART) to download saved data and perform diagnostic functions, a UART connected detect circuit, and an LED connected to the UART transmit.

In some embodiments, the pH probe circuit is designed without amplifiers, but instead limits the positive and negative references for the analogue digital converter in the microcontroller to the appropriate the range of output voltage. The reference voltage of the sensor is offset with a simple resistor divider network. A processor is required which includes a voltage reference which can be output, and an analogue digital converter. In using fewer components compared with a more conventional design, the pH probe more easily fits into a small space. A reduced circuit with a simple resistor divider network in place of amplifiers represents a suitable approach for the requirements of the sampling device.

Typically, a pH sensor (69) is dependent on the pH condition in which it is embedded, which can be preprogrammed to react/ respond to a certain pH condition depending on the external environment in which the device is placed, for example the location along the gastrointestinal tract and/or a tank (i.e. for bioprocessing/fish/other processing etc) and/or other agricultural systems. The pH sensor can be preprogrammed to activate at pH levels of 1, 2, 3, 4, 5, 6, 7, 8, 9, 10, 11, 12, 13 or 14 and/or any ranges and/or combinations thereof. For example, a pH below 4 can be preprogrammed so that the device is activated to its second configuration (e.g. in the stomach), or preprogrammed at pH above 5 so that the device is activated to its second configuration (e.g. in the small or large intestines and/or just upon entry to the small or large intestine). The activation may include a temporal element such that activation is delayed for a predetermined time following detection of a specific pH level. The activation may also be programmed such that a consistency of pH level is required before activation is triggered. For example, so that the retention means is not released.

In particular, the glass electrode may protrude from the surface of the separate compartment, so that it is exposed to the external environment of the sampling device to be capable of measuring the pH at an accuracy of +/-0.5 pH units (5% accuracy) and able to take multiple readings (for example about 3000 readings or more), which can be preconfigured to determine the rate the pH is measured and logged.

In particular, the sensor/meter may be connected to the battery (62) and the associated electronic circuits within the separate compartment (30).

Further, the circuit can also log the actuation moment at which point the sample is drawn into the housing (i.e. collected). This is achieved by monitoring the release of the switch (i.e. when the switch is open and no longer in contact and/or engaged with the actuation means).

In some embodiments, the device may further include a temperature sensor, a pressure sensor, an ultra sound sensor, a biosensor and/or a solute sensor, or the like.

In some embodiments, the sensor/meter can be programmed to release the retention means relative to a particular pH unit, timing, temperature solute concentration or any combinations of these parameters. For example, such programming may include: a timing of 60 minutes after a pH increases about pH3 or pH increases about 2pH units and remains elevated for 3 consecutive minutes, etc.

In some embodiments, the sampling device further comprises a retention means (100) which releasable retains the separate compartment (30) within the housing (20) of the sampling device. In particular, the housing of the sampling device includes a retention means.

The retention means (100) temporarily prevents the actuation means (50) (e.g. plunger/piston) from ejecting from the housing until a predetermined time and/or condition. The retention means can be any element or object or mechanism which detains the separate compartment (30) from being released from the housing of the device. In particular, the retention means holds the separate compartment within the housing of the device and maintains the device in a compressed and closed configuration.

The retention means can be can be passively or actively activated.

In particular, the retention means can be activated automatically, preprogrammed and/or activated remotely.

In some embodiments, the retention means (100) can be a material that reacts to changes in the external environment of the device and/or an interlocking mechanism and/or a fastening means.

In some embodiments, the retention means (100) can be a material that reacts to changes in the external environment of the device. The retention means can be in the form of a coating, pin and/or washer. In preferred embodiments, the retention means is in the form of a coating. The coating may surround the entire device or may partially cover only certain parts of the device or be located at a particular part of the device.

Typically, the device can encounter differences in physiological characteristics, such as pH, pressure, temperature, enzymatic activity, etc (e.g. depending on whether the device is in the stomach, the colon, etc., especially when travelling along the gastrointestinal tract of an animal). The external environment of the device is therefore variable. In particular embodiments, the sampling device can comprise a retention means (100) that is a material that reacts to pH, temperature, light, moisture, solute concentration or enzyme activity or concentration. In particular, the material can be degradable, digestible or soluble.

The material may surround the entire device or may partially cover only certain parts of the device or be located at a particular part of the device. The material may be located and/or cover the end of the device which ejects the separate compartment (30). The material may also cover the opening, for example the inlet, outlet and/or inlet and outlet. In particular, the material may be in the form of a coating, pin and/or washer.

In some embodiments, the retention means (100) can be a material that reacts to changes in the external environment, wherein the retention means is a material in the form of a coating. The coating may surround the entire device and/or parts of the device. The coating aids the device to retain the separate compartment (30) within the housing (20).

In some embodiments, the retention means (100) can be a material that reacts to changes in the external environment, wherein the retention means is a material in the form of a washer. The washer aids to retain the separate compartment (30) with the housing (20).

In some embodiments, the retention means (100) can be a material that reacts to changes in the external environment, wherein the retention means is a material that can be in the form of a coating and a washer, thereby retaining the separate compartment within the housing and also maintaining the washer dry and keeping the mechanical strength of the washer intact.

The material dissolves gradually upon contact with a change in pH, a change in temperature, a change in light, a change in moisture, a change in solute concentration or enzymatic environment. The rate of degradation, digestibility and or solubility may be controlled by either differences in the chemical structure of the material or by differences in the thickness of the application and/or form of similar materials, or both. In particular, the material may require two or more layers of the same material or of different materials.

In some embodiments, the pH-sensitive material may comprise and/or consist of a pH-sensitive material that degrades in an alkaline environment or in an acidic environment. The rate of degradation may be controlled by either differences in the chemical structure of the material or by differences in the thickness application and/or form of similar materials, or both.

In some embodiments, a first material/layer is dissolved in response to a first pH, first temperature, first wavelength of light, first soluble concentration or first enzymatic activity, and a second material/layer is dissolved in response to a second, different pH, different temperature, different wavelength of light, different solute concentration or different enzymatic activity.

The material can be pH dependent and dissolve in the stomach when in contact with gastric acid and in acidic conditions under pH 4 or in the large intestine when in alkaline conditions such as a pH 5 to 6 or above 7, and/or any combinations thereof.

In particular embodiments, the material can be cellulose, acetate phthalate, glycerol stearates, paraffin, epoxy compounds or poly (methyl) acrylates, such as Eudragit® L, S or E. Preferably, the material is Eudragit® L, S or E.

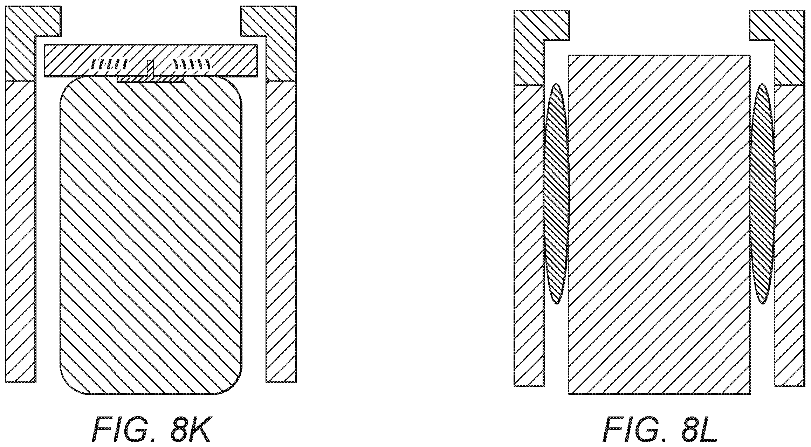

In some embodiments, the material may comprise and/or consist of a temperature sensitive material that degrades upon an increase or decrease in temperature. The heat-sensitive material may be glue, glue like material or wax. In particular, the wax may be paraffin wax, microcrystalline wax, ester wax, polyester wax or vegetable wax. The preferred embodiment can be paraffin wax with a melting point above the body temperature of the animal. Figure 8K represents an example of a thermal sensitive material as a retention means (100).

In some embodiments, the material may comprise and/or consist of a photo-sensitive material that degrades upon a certain wavelength, such as glue and/or other alike materials known in the art. In particular, when the material is exposed to light, for example being exposed to a light emitting diode, the (glue) material degrades. Figure 8L represents an example of a photo-sensitive material as a retention means (100).

In some embodiments, the material may comprise and/or consist of material that degrades upon a change in moisture of solute concentration.

In some embodiments, the material may comprise and/or consist of material that degrades upon a change in enzyme activity and/or concentration.

In alternative embodiments, the retention means (100) can be an interlocking mechanism. In particular the interlocking mechanism can be between the housing (20) and the separate compartment (30). In some embodiments, the retention means may comprise a material that reacts to changes in the external environment of the device and an interlocking mechanism. The material that reacts to changes in the external environment may react to pH, temperature, light, moisture, solute concentration or enzyme activity or concentration. In particular, the material can be degradable, digestible or soluble.

The material may surround the entire device or may partially cover only certain parts of the device or be located at a particular part of the device. The material may be located and/or cover the end of the device which ejects the separate compartment (30). The material may also cover the opening (44), for example the inlet, outlet and/or inlet and outlet. In particular, the material may be in the form of a coating, pin and/or washer. The material may be in the form of a coating.

The material dissolves gradually upon contact with a change in pH, a change in temperature, a change in light, a change in moisture, a change in solute concentration or enzymatic environment. The rate of degradation, digestibility and or solubility may be controlled by either differences in the chemical structure of the material or by differences in the thickness of the application and/or form of similar materials, or both. In particular, the material may require two or more layers of the same material or of different materials.

In some embodiments, the pH-sensitive material may comprise and/or consist of a pH-sensitive material that degrades in an alkaline environment or in an acidic environment. The rate of degradation may be controlled by either differences in the chemical structure of the material or by differences in the thickness application and/or form of similar materials, or both.

In some embodiments, a first material/layer is dissolved in response to a first pH, first temperature, first wavelength of light, first soluble concentration or first enzymatic activity, and a second material/layer is dissolved in response to a second, different pH, different temperature, different wavelength of light, different solute concentration or different enzymatic activity.

The material can be pH dependent and dissolve in the stomach when in contact with gastric acid and in acidic conditions under pH 4 or in the large intestine when in alkaline conditions such as a pH 5 to 6 or above 7, and/or any combinations thereof.

In particular embodiments, the material can be cellulose, acetate phthalate, glycerol stearates, paraffin, epoxy compounds or poly (methyl) acrylates, such as Eudragit® L, S or E. Preferably, the material is Eudragit® L, S or E.

In some embodiments, the material may comprise and/or consist of a temperature sensitive material that degrades upon an increase or decrease in temperature. The heat-sensitive material may be glue, glue like material or wax. In particular, the wax may be paraffin wax, microcrystalline wax, ester wax, polyester wax or vegetable wax. The preferred embodiment can be paraffin wax with a melting point above the body temperature of the animal. Figure 8K represents an example of a thermal sensitive material as a retention means (100).

In some embodiments, the material may comprise and/or consist of a photo-sensitive material that degrades upon a certain wavelength, such as glue and/or other alike materials known in the art. In particular, when the material is exposed to light, for example being exposed to a light emitting diode, the (glue) material degrades. Figure 8L represents an example of a photo-sensitive material as a retention means (100).

In some embodiments, the material may comprise and/or consist of material that degrades upon a change in moisture of solute concentration.

In some embodiments, the material may comprise and/or consist of material that degrades upon a change in enzyme activity and/or concentration.

Typically, an interlocking mechanism is a mechanical element allowing coupling of one or more elements which is/are capable of affecting the separate elements and/or other objects in motion or operation.

In particular, the interlocking mechanism can be fastened or released by rotary, radial or linear motion or means.

In particular, the retaining means (100) can be a (i) rotational (i.e. rotary) interlocking mechanism or (ii) a radial interlocking mechanism or (iii) a linear interlocking mechanism.

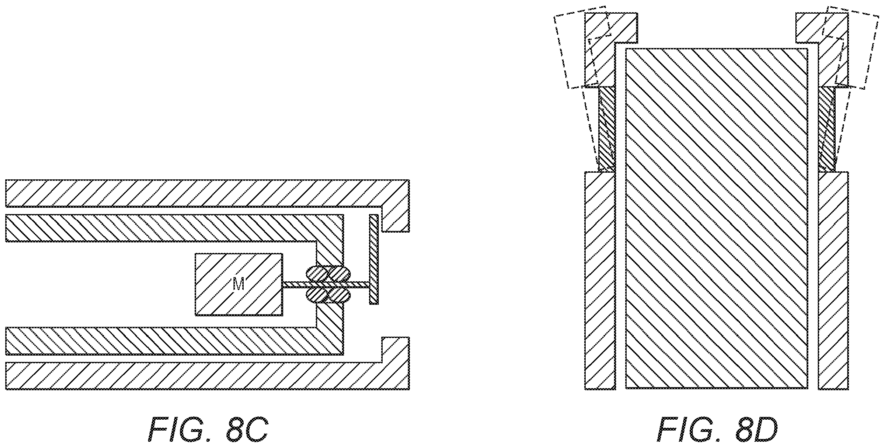

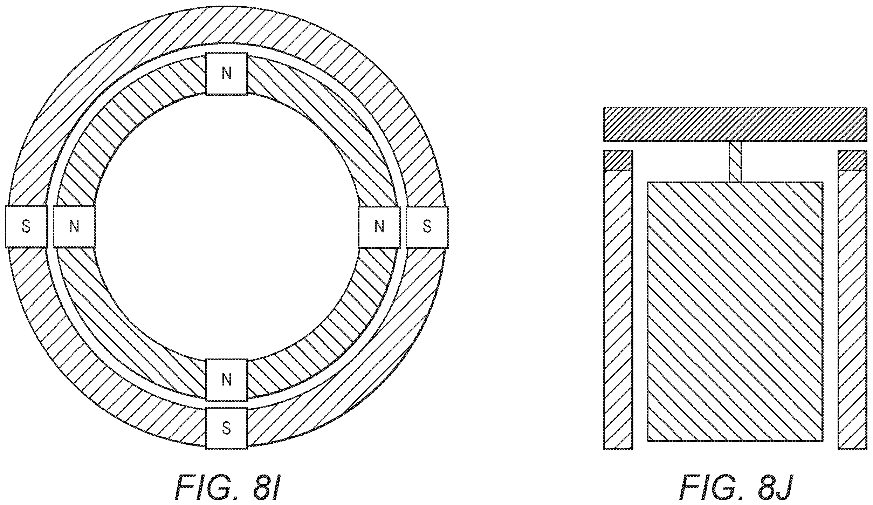

In some embodiments, the interlocking mechanism may be rotary, such as a bayonet mount (100A), a rotating point catch, a set of rotating magnets or electromagnets (see representations of these in Figures 8C and 8I). Preferably, the rotary interlocking mechanism is a bayonet mount.

Bayonet mounts are typically known in the art as fastening elements which consist of a male side with one or more radial pins, and a female receptor with matching L-shaped slot(s) and with spring(s) (101) to keep the two parts engaged and locked together. The slots may be shaped like a capital letter L with serif (a short upward segment at the end of the horizontal arm); the pin slides into the vertical arm of the L, rotates across the horizontal arm, then is pushed slightly upwards into the short vertical "serif" by the spring; the connector is no longer free to rotate unless pushed down against the spring until the pin is out of the "serif" and therefore unlocked and released.

In particular, wherein the retention means can be a bayonet mount, the actuation means (50) (e.g. the plunger/piston) is attached into the device opening (44) such that it cannot rotate when in the compressed condition. The separate compartment (30) is made to pivot against this attachment until the bayonet mount interlocking mechanism (100A) is aligned in such a way that the separate compartment is no longer retained and thus the separate compartment is released. Thereby, releasing the pressure in the housing (30) of the device and thus the actuation means is expanded along the housing pushing the separate compartment out of the device from the first compressed configuration to the second expanded configuration.

In particular embodiments, the rotary mechanism can be a rotating point catch. In particular, a pin can interfere with the stopper means on the housing (20) of the device. On activation, the device is rotated by a driving force such that it clears the stopper means and the separate compartment (30) is released. Figure 8C represents an example of a rotating point catch interlocking mechanism.

In particular embodiments, the rotary mechanism can be a set of rotating magnets. In particular, permanent magnets are included in the wall of the housing and separate compartment. When held in alignment, the attraction of the magnets holds the separate compartment (30) within the housing (20) of the device. When exposed to a rotating force, the magnets are moved out of alignment and the force of attraction is lost. The separate compartment is then released. Figure 8I represents an example of rotating magnets as a rotary interlocking mechanism.

In particular embodiments, the rotary mechanism can be electromagnetic. In particular, electromagnets embedded into the walls of the separate compartment (30) and the housing (20) are energised such that they are attracted. This attraction is sufficient to prevent the separate compartment from moving against the housing. On removal of the energising current, the two compartments are no longer held by an attractive force and the separate compartment is then released.

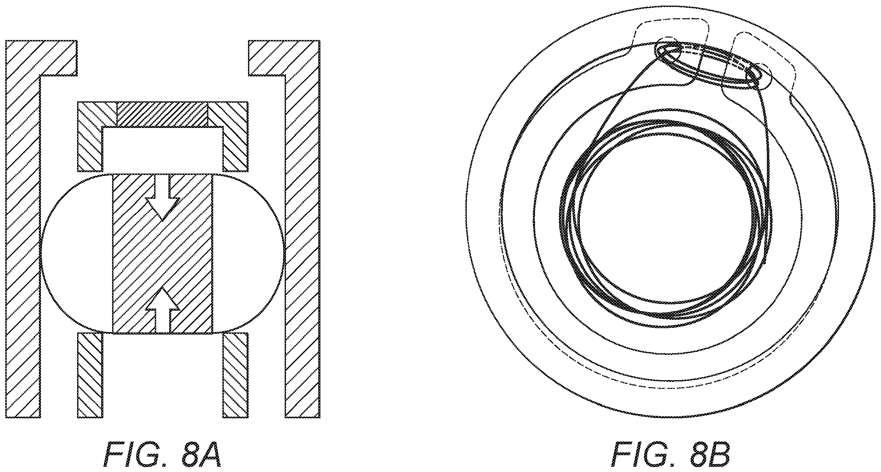

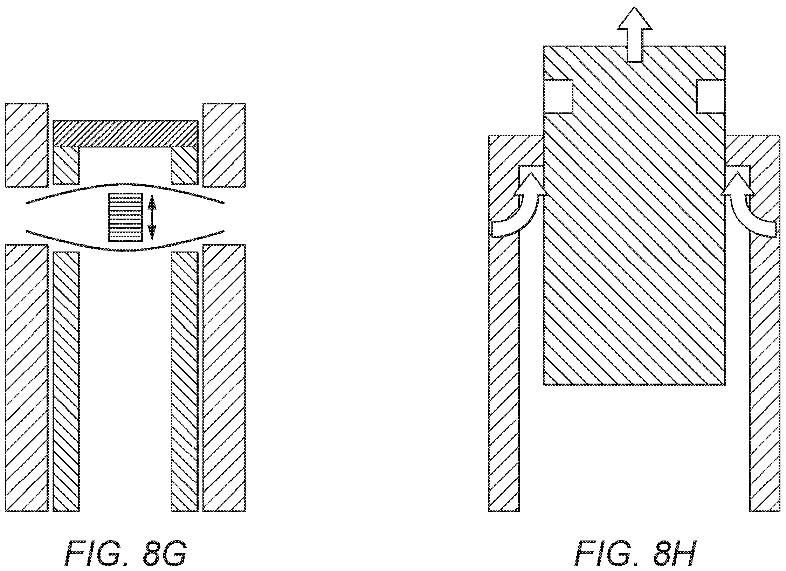

In some embodiments, the interlocking mechanism can be radial, such as a compressible material (i.e. a rubber O-ring), bowed member friction, a circlip or a pull in catch (see representations of these in Figures 8A, 8B or 8G). Preferably, the radial interlocking mechanism is a compressible material such as rubber O-ring washer (100B).

In particular embodiments, the radial interlocking mechanism can be any compressible material. In some embodiments, the material may be elastomeric. In particular, the compressible material may be an O-ring washer (100B) or a bending strip element. In particular, the ring of compressible material is compressed between the lid (34) and body (32) of the separate compartment (30). This compression causes the material of the O-ring to expand outwards (radially) such that it comes into contact with the inner face of the housing (20) of the device. The resulting friction is sufficient to prevent the separate compartment from being ejected and therefore being releasably retained. The compression of the ring is reduced at a particular condition and/or predetermined time, reducing the friction between the separate compartment and the housing and thereby releasing the separate compartment.

In particular embodiments, the radial interlocking mechanism can be a bowed member. In particular, longitudinal elements on the external surface of the separate compartment (30) are held in compression such that they are caused to bow out. These elements come into contact with the inner surface of the housing (20) of the device, causing a frictional interference that prevents the separate compartment from being released. At a particular condition and/or predetermined time, compression of the longitudinal elements is reduced, reducing the friction between the separate compartment and the housing, allowing the separate compartment to be released. Figure 8A represents an example of a bowed member radial interlocking mechanism.

In particular embodiments, the radial mechanism can be a circlip. Typically, a circlip is a form of fastener which consists of a semi-flexible metal ring with open ends, which can permit rotation but prevent lateral movement. In particular, the circlip is fitted to a groove on the outer surface of the separate compartment (30). The circlip may interfere with the stopper means of the housing (20) of the device. The circlip is squeezed together at a particular condition and/or predetermined time, reducing its diameter and allowing it to move past stopper means, allowing the separate compartment to be released. Figure 8B represents an example of a circlip radial interlocking mechanism.

In particular embodiments, the radial interlocking mechanism can be a pull-in catch. In particular, a strip of material is deformed such that it protrudes through apertures on the side of the separate compartment (30) and engages with features on the inside wall of the housing (20) of the device. These features interfere with the strip to prevent the separate compartment from being released. At a particular condition and/or predetermined time, the strip relaxes and retreats from these features, allowing the separate compartment to be released. Figure 8G represent an example of a pull-in catch as a radial interlocking mechanism.

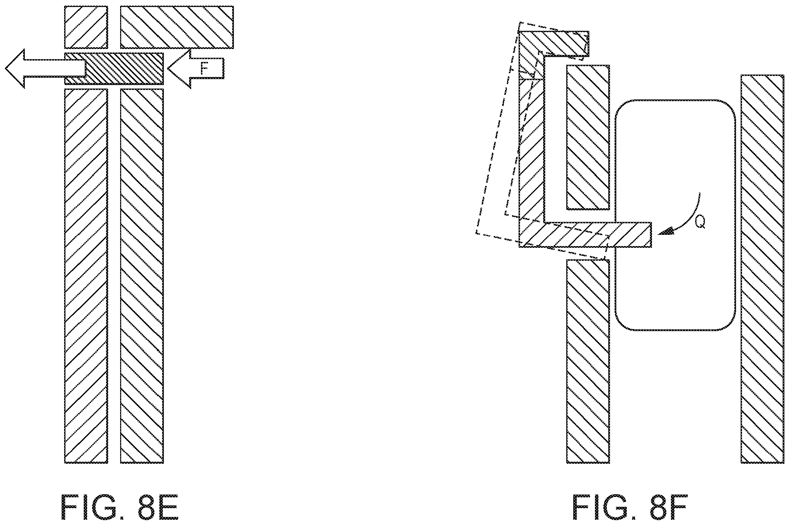

In some embodiments, the interlocking mechanism may be linear, such as a deformable lip and/or barrel, tab or pin at the end of the housing (20) or pin that holds the separate compartment (30) into place or a bi-metallic latch that protrudes through the housing holding the separate compartment into place, electromagnets and/or a shape memory alloy (see representation of these in Figures 8D, 8E, 8F, 8H or 8J). Preferably, the linear interlocking mechanism is a pin that pushes the separate compartment into place.

In particular embodiments, the linear interlocking mechanism can be a deformable lip. In particular, a region of the housing (20) is made deformable, such that when pushed against, the separate compartment (30) is released. Typically, cuts (e.g. lips) are made into the housing of the device, such that it becomes more easily deformed. In particular, such cuts are made of deformable material such as a shape memory alloy. At a particular condition and/or predetermined time, the separate compartment is pushed against the cuts, which fold back to allow the separate compartment to be released and then return to its normal state to stop the actuation means (50) from being released. Such cuts can also function as the stopper means. Figure 8H represents an example of a deformable pin as a linear interlocking mechanism.

In particular embodiments, the linear interlocking mechanism can be a shape memory alloy (SMA). In particular, a shaped element consisting of a shape memory alloy is deformed, such that it interferes between the separate compartment (30) and the housing (20). At a particular condition and/or predetermined time, a current may be applied, or the shape memory alloy may be heated by some other means, the shape memory alloy reverts to its original shape. The original shape is specified, such that it allows the separate compartment to be released. Figure 8H represents an example of a shape memory alloy as a linear interlocking mechanism.

In particular embodiments, the linear interlocking mechanism can be a pin. In particular, a pin is located between a hole in the wall of the separate compartment (30) and the housing (20) of the device, preventing the separate compartment from being released. At a particular condition and/or predetermined time, the pin is either pulled inwards or pushed outwards, allowing the separate compartment to be released. Figure 8E represent an example of a pin as a linear interlocking mechanism.

In particular embodiments, the linear interlocking mechanism is a bi-metallic latch. In particular, a shaped bi-metallic strip is fixed to the outer surface of the housing (20), such that one end protrudes through a hole in the housing and into a hole in the separate compartment (30). At a particular condition and/or predetermined time, the bi-metallic strip is heated and thereby deforms, causing the protruding end to retreat and allowing the separate compartment to be released. Figure 8F represent an example of a bi-metallic latch as a linear interlocking mechanism.

In particular embodiments, the linear interlocking mechanism can be electromagnets. In particular, the end surface of the separate compartment (30) consists of a ferrous material. The end of the housing (20) is made from a ferrous electromagnet. A current is passed through the electromagnet, generating an attractive force between the end of the housing and the end surface of the separate compartment, preventing the separate compartment from being released. When the current is removed, the attractive force no longer exists and the separate compartment can be released. Figure 8J represents an example of electromagnets as a linear interlocking mechanism.

In yet other embodiments, the retention means (100) can be a fastening means and/or a material that reacts to changes in the external environment of the device (as previously described). In particular, the actuation means (50) of the device is tethered to a closure means (80) and wherein the movement of the actuation means results in the closure means blocking the opening. In other embodiments, the fastening means is also tethered to the closure means.

A fastening means can be any device or coupled elements, which can hold and secure other objects to prevent movement or separation through the application of pressure.

In particular, the fastening means can be a clamp which holds the tether (82) in place under tension.

In particular embodiments, the fastening means can be activated by any form of interlocking mechanism previously described. The interlocking mechanism may be fastened or released by rotary, radial or linear motion or means. Preferably, the interlocking mechanism is a catch. Any form of catch can be used known in the art.

The catch may consist of a number of parts that are caused to interfere with each other, possibly in association with levers and pivot points. The parts may include hook elements and/or friction elements. One part of this grouping can be flexible, such that when a force is applied to it, it bends or deforms or otherwise moves. This movement is arranged such that the constituent parts of the catch no longer interfere with each other and the catch is released, thereby releasing the retention means and thus releasing the separate compartment.

In this particular embodiment, the actuation means (50) may not comprise a spring, may not comprise a valve and may not comprise a stopper means.

In this particular embodiment, the actuation means (50) (e.g. the plunger/piston) and the closure means (80) are tethered. When the retention means (100) (either the fastening means and/or the material that reacts to changes in the external environment of the device) is activated (passively or actively), the separate compartment (30) is released, thereby the actuation means moves along the housing (20) of the sampling device due to a pressure drop within the housing of the device. The actuation means tethered with the closure means is under tension. The actuation means moves along the housing of the device, the tensed tether (82) pulls the closure means along until the closure means reaches the opening. The closure means is pushed or pulled into the opening (44), forming a closed device. Simultaneously, the actuation means draws up sample into the chamber of the device. Thereby, the sample is sealed within the device. As the actuation means is tethered to the closure means under tension, the actuation means (plunger/piston) cannot be released further and thus a stopper means is not required to stop the plunger from exiting from the device.

In alternative embodiments, the retention means (100) is also tethered to the closure means (80) under tension.

Further embodiments, may comprise a spring (52) to increase the force in which the actuation means (50) drives along the housing.

The sampling device may further comprise a trigger means (70). The trigger means can be an element of the device, which can be activated automatically, pre-programmed and/or activated remotely. A trigger means can be an element which is capable of interaction with the retention means (100) to provide the effect to release the separate compartment (30) of the sampling device.

In particular embodiments, the retention means (100) can be activated by a trigger means (70) and/or in response to a release parameter.

In particular embodiments, the interlocking mechanism can be activated by a trigger means (70) and/or in response to a release parameter.

In particular embodiments, the fastening means can be activated by a trigger means (70) and/or in response to a release parameter.

In particular, the trigger means (70) can be activated automatically, preprogramed and/or activated remotely.

In some embodiments, the trigger means (70) may be a form of electronic actuation.

Electronic actuation can be selected from electro-magnetic means (such as solenoids and/or motor, magnetostrictive materials), piezo-electronic means (such a stack or diaphragm), shape changers (muscle wire), electro-chemical (battery gassing, spark generator, light sensitive adhesive), heating element and/or fusing (fuse blowing, heating element or wax actuator).

In particular embodiments, the trigger means (70) can be a solenoid or a motor. The solenoid or motor may consist of a wire coiled around a central metallic core. When a current is passed through the wire, the resulting electromagnetic field causes the solid core to move. This movement can be utilised to effect one or more of the mechanisms required to release the interlocking mechanism and/or retention means (100).

In particular embodiments, the trigger means (70) can be a magnetorestrictive material. When a magnetic field is applied to the magnetorestrictive material, the material changes shape and/or dimension. This change in shape or dimension can be utilised as a kinetic energy source to affect one or more of the mechanisms required to release the interlocking mechanism and/or retention means.

In particular embodiments, the trigger means (70) can be a piezomagnetic material. When an electrical field is applied to a piezomagnetic material there is a resulting mechanical strain. The force produced by this piezoelectric effect can be utilised as a kinetic energy source to affect one or more of the mechanisms required to release the interlocking mechanism and/or retention means. The available force may be multiplied by stacking piezo electric elements.

In particular embodiments, the trigger means (70) can be a muscle wire. In particular, a muscle wire can be a fibre of nickel-titanium alloy (e.g. Nitinol® or Flexinol®) that changes length in the presence of an electric current. The force generated by this change in length can be utilised as a kinetic energy source to effect one or more of the mechanisms required to release the interlocking mechanism and/or retention means (100).

In particular embodiments, the trigger means (70) can be a fuse. In particular, a length of material is used to withhold the energy of the actuation means (50) (e.g. comprising a spring), preventing the separate compartment (30) from being released from the housing (20) of the device. When a current is applied to the material, electrical resistance causes the material to heat up and mechanically fail. Typically, the material would be zinc, copper, silver, aluminum or alloys designed to provide a predictable failure characteristic. Failure of the fuse would release the interlocking mechanism and/or retention means allowing the separate compartment to be released.

In further embodiments, the retention means (100) and/or the trigger means (70) can be activated automatically, pre-programmed and/or activated remotely.

The trigger means (70) may be initiated automatically in response to a release parameter (pH, temp, etc.). The trigger means can be initiated by instructions programmed into the microprocessor of the separate compartment (30) in response to a release parameter. The trigger means can also be initiated by an external wireless system in response to an external release parameter e.g. time, daylight, or through direct intervention by a user of the system.

In some embodiments, the retention means (100) and/or the trigger means (70) are activated in response to a release parameter. In particular embodiments, the retention means is released in response to a release parameter.

When the release parameter can be activated by physiological characteristics, such as pH, pressure, temperature, or external remote activation, the release parameter may activate the retention means (100) and/or trigger means (70).

A release parameter can be a change in pH, a change in temperature, a change in light, a change in moisture, solute concentration and/or enzyme activity or concentration and/or can be activated at a predetermined time and/or at a predetermined pH and/or at a predetermined temperature and/or predetermined light, and/or predetermined moisture and/or predetermined solute concentration and/or predetermined enzyme activity or concentration or location and/or environmental conditions, etc.

In particular embodiments, the release parameter can be activated remotely from outside the body of the animal, or can be pre-programmed based on time, or can be activated based on other physiological characteristics, such as pH, pressure, temperature, etc.

In particular, the release parameter may be responsive to the external environment of the device and/or activated in response to a predetermined signal from the internal processor and/or controller in the device.

The device can be programmed to open relative to a pH signal that signifies gastric emptying, for example, 60 minutes after pH increases above pH3 or 60 minutes after pH has risen for at least 2 pH units and remained elevated for 3 consecutive minutes. Additionally, the change in temperature can also be useful to identify elevations in gastric pH due to ingestion of water, so that these instances are not mistaken for gastric emptying.

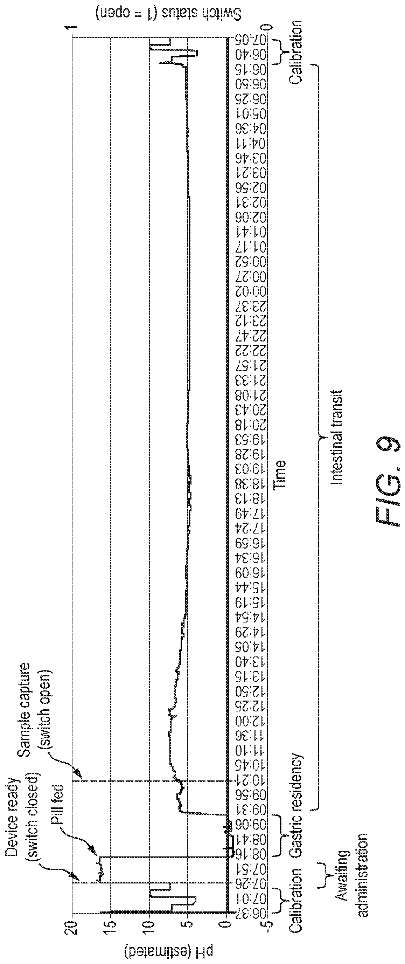

Calibration techniques using parameters such as time, temperature, pH, etc. to determine location in the gastrointestinal tract of an animal are known in the art. In general, the pH during transit in the gastrointestinal tract would be expected to rise sharply on gastric emptying, continue to rise at a slower rate along the small intestine, drop sharply on entering the large intestine, before starting to rise again very slowly (this can be seen in Figure 9). In particular, the timing at which the device reaches the end of the small intestine may be identified, for example, as follows; (1) detecting a rise in pH of at least 4 pH units, (2) such rise in pH persists for about 10 minutes and (3) such rise is at least 30 minutes after exiting the stomach.

In some embodiments, the device may include a controller (internally or externally from the device) which controls the activation of the release parameter. In particular, the controller may either generate a signal at a predetermined time or may receive a signal externally or generate a signal in response to sensed parameters, such as pH, temperature, pressure, enzyme activity, or the like.

In some embodiments, a signal is induced in the circuit of the device by an array of electromagnetic coils external to the body of the test subject. Movement of the test subject, (for example by walking between the array of electromagnetic coils, or by moving a handheld coil array around the body of the test subject), induces a current in the circuit of the device. This current is interpreted as a signal that functions as a release parameter.

In some embodiments, the circuit contains a signal receiving coil that receives a wireless signal from an external transmitter. This signal can be initiated at any time by an external operator and functions as the release parameter.

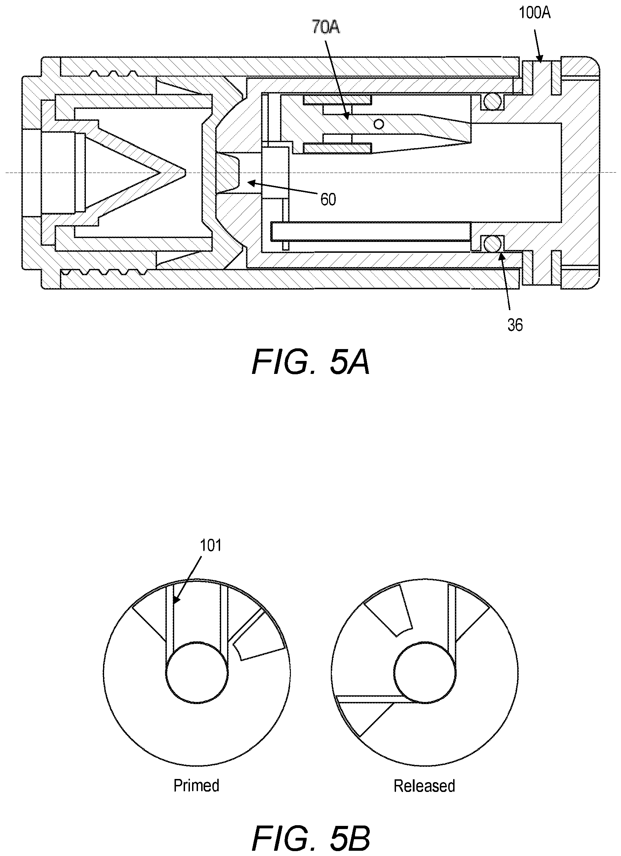

In particular specific embodiments of the invention, the retention means (100) can be a rotary interlocking mechanism, such as bayonet mount (100A), that is activated by a trigger means (70A). Figures 5A and 5B are schematic illustrations of an example of a bayonet mount as a rotary interlocking mechanism on the device.

In specific embodiments of the invention, the retention means can be a rotary interlocking mechanism, such as a bayonet mount (100A), that is activated by a trigger means (70A), such as a piezo beam.

In particular, the separate compartment (30) is inserted into the housing (20) and is rotated to engage with the actuation means (50). The separate compartment and the actuation means (i.e. plunger/piston) engage with each other in order to maintain a stored energy by means of a pin or other locating device. This rotation causes a spring (101) in the interlocking mechanism, which provides the force to rotate the separate compartment within the housing, to be extended, storing an amount of energy within the spring. The rotation also causes protrusions on the separate compartment and housing to be aligned, preventing the separate compartment from being released and thereby releasably retained. The device is thereby in its compressed configuration.

When the sensors (e.g. pH and/or temperature) detect an appropriate change in pH and/or temperature, a signal is sent from the microprocessor (64) to a piezo electric beam, causing the beam to be deflected. This deflection disengages the locating pin and allows the stored spring energy to be released, rotating the separate compartment (30) within the housing (20). This rotation misaligns the protrusions in the separate compartment and the housing, allowing the separate compartment to be ejected by the actuation means.

In specific embodiments of the invention, the retention means can be a rotary interlocking mechanism, such as a bayonet mount, that is activated by a trigger means, such as a muscle wire.

In particular, the separate compartment (30) is inserted into the housing (20) and is rotated to engage with the actuation means (50) by a pin or other locating device. This rotation causes a spring (101) to be extended, storing an amount of energy within the spring. The rotation also causes protrusions on the separate compartment and housing to be aligned, preventing the separate compartment from being released. The device is now in its compressed state. When the sensors (e.g. pH and/or temperature) detect an appropriate change in pH and/or temperature, a signal is sent from the microprocessor to a length of muscle wire, causing the muscle wire to shorten. One end of the muscle wire is attached to the locating pin. This shortening disengages the locating pin and allows the stored spring energy to be released, rotating the separate compartment within the housing. This rotation misaligns the protrusions in the separate compartment and the housing, allowing the separate compartment to be ejected by the actuation means.

In specific embodiments of the invention, the retention means can be a rotary interlocking mechanism, such as a bayonet mount (100A), that is activated by a trigger means (70), such as a shape memory alloy (SMA).

In particular, the separate compartment (30) is inserted into the housing (20) and is rotated to engage with the actuation means (50) by a pin or other locating device. This rotation causes a simple spring (101) element to be extended, storing an amount of energy within the spring. The rotation also causes protrusions on the separate compartment and housing to be aligned, preventing the separate compartment from being released. The device is now in its compressed state. When the sensors (e.g. pH and/or temperature) detect an appropriate change in pH and/or temperature, a signal is sent from the microprocessor to a length of deformed shape memory alloy, causing the shape memory alloy to revert to its initial form. One end of the shape memory alloy is attached to the locating pin. This change in form disengages the locating pin and allows the stored spring energy to be released, rotating the separate compartment within the housing. This rotation misaligns the protrusions in the separate compartment and the housing, allowing the separate compartment to be ejected by the actuation means.

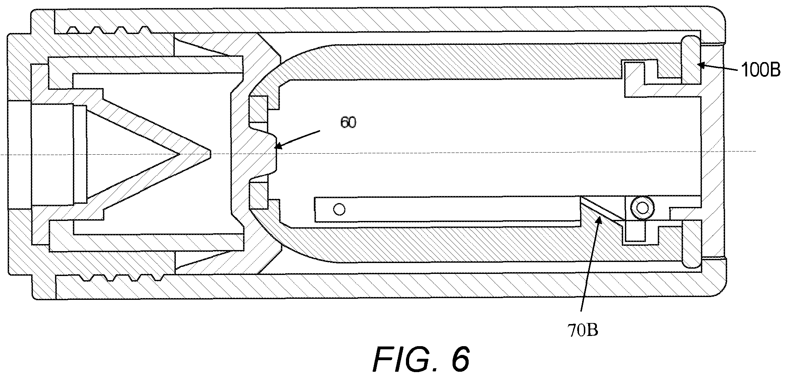

In particular specific embodiments of the invention, the retention means (100) can be a radial interlocking mechanism, such as compressible material that is activated by a trigger means (70B). Figure 6 is a schematic illustration of an example of an O-ring (100B) as a radial interlocking mechanism on the device.

In specific embodiments of the invention, the retention means can be a radial interlocking mechanism, such as a compressible material in the form of an O-ring, that is activated by the trigger means such a piezo beam.

In particular, the separate compartment (30) is inserted linearly into the housing (20). Continuing linear force on the separate compartment (after it is inside the housing) causes an elastic O-ring (100B) to be compressed between the cap (34) and body (32) of the separate compartment. This compression causes deformation of the O-ring, such that it interferes with a stopper means (e.g. lip) on the end of the housing, preventing the separate compartment from being released. The O-ring is kept in compression by a catch mechanism within the separate compartment. When the sensors (e.g. pH and/or temperature) detect an appropriate change in pH and/or temperature, a signal is sent from the microprocessor to a piezo electric beam, causing the beam to be deflected. This deflection disengages the catch mechanism and allows the O-ring to be restored to its initial, decompressed state in which it no longer interferes with the stopper means (e.g. lip) on the housing. The separate compartment is then ejected by the actuation means (50).

In specific embodiments of the invention, the retention means can be a radial interlocking mechanism, such as a compressible material in the form of an O ring, that is activated by the trigger means such a Muscle Wire.

In particular, the separate compartment (30) is inserted linearly into the main body of the pill. Continuing linear force on the separate compartment (after it is inside the main pill body) causes an elastic O- ring (100B) to be compressed between the cap (34) and body (32) of the separate compartment. This compression causes deformation of the O-ring such that it interferes with a lip on the end of the main pill body, preventing the separate compartment from being ejected. The O-ring is kept in compression by a catch mechanism within the separate compartment. When the sensors (e.g. pH and/or temperature) detect an appropriate change in pH and/or temperature a signal is sent from the microprocessor to a length of muscle wire, causing the muscle wire to shorten. One end of the muscle wire is attached to the catch mechanism. This shortening disengages the catch mechanism and allows the O-ring to be restored to its initial, decompressed state in which it no longer interferes with the lip on the main body of the pill. The separate compartment is then free to be ejected by the actuation means (50).

In specific embodiments of the invention, the retention means can be a radial interlocking mechanism, such as a compressible material in the form of an O-ring, that is activated by the trigger means such a Shape Memory Alloy (SMA).

In particular, the separate compartment (30) is inserted linearly into the housing (20). Continuing linear force on the separate compartment (after it is inside the housing) causes an elastic O-ring (100B) to be compressed between the cap (34) and body (32) of the separate compartment. This compression causes deformation of the O-ring, such that it interferes with a stopper means (e.g. lip) on the end of the housing, preventing the separate compartment from being released. The O-ring is kept in compression by a catch mechanism within the separate compartment. When the sensors (e.g. pH and/or temperature) detect an appropriate change in pH and/or temperature, a signal is sent from the microprocessor to a length of deformed shape memory alloy, causing the shape memory alloy to revert to its initial form. One end of the shape memory alloy is attached to the catch mechanism. This change in form disengages the catch mechanism and allows the O-ring to be restored to its initial, decompressed state in which it no longer interferes with the stopper means on the housing. The separate compartment is then free to be ejected by the actuation means (50).

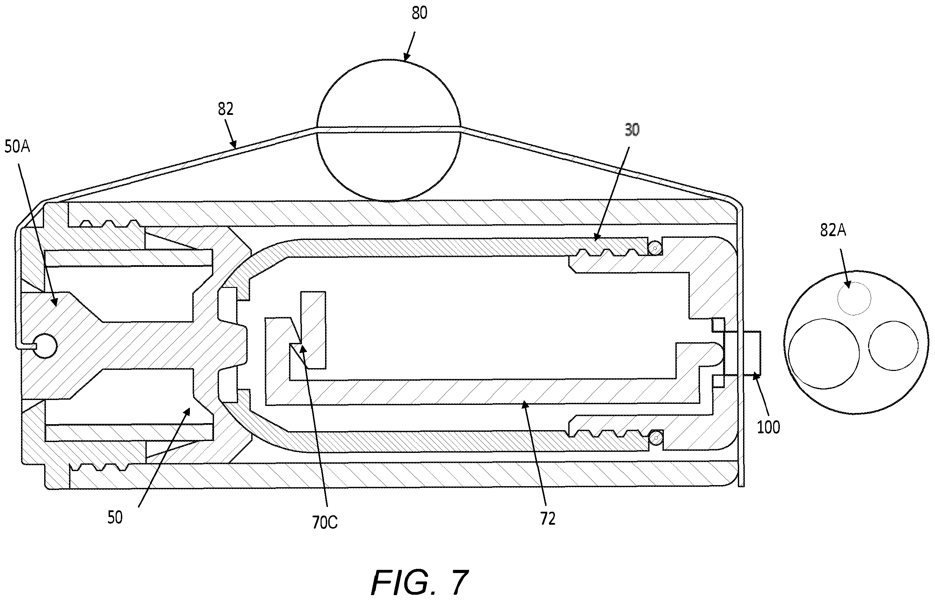

In particular, specific embodiment of the invention, the retention means (100) can be a fastening means that is activated by a trigger means. Figure 7 is a schematic representation of an example of a fastening means as a retention means (100) of the device.

In specific embodiments of the invention, the retention means can be a fastening means that is activated by a trigger means, such as a piezo beam.

In particular, the separate compartment (30) is inserted linearly into the housing (20), causing the actuation means (50) to be compressed. A tether (82) is attached to the actuation means and extends around the outer surface of the device and is gripped in by the fastening retention means (100) (such as a clamping mechanism). The fastening means consists of a protrusion extending through a membrane on the surface of the separate compartment. The protrusion is held in position by a latch and lever mechanism (70C) inside the separate compartment. The latch may be initially engaged by the temporary action of a magnet or electromagnet. The fastening means allows the tie to be kept under tension (as in 82A), thereby preventing movement of the actuation means (50). When the sensors (e.g. pH and/or temperature) detect an appropriate change in pH and/or temperature, a signal is sent from the microprocessor to a piezo electric beam (72), causing the beam to be deflected. This deflection disengages the catch and lever mechanism (70C) and allows the fastening means to release. The actuation means is then free to push and eject the separate compartment. As the actuation means travels along the housing, the tether (82) is drawn though the opening pill. A closure means (80) is attached at a point on the tether that serves to block the opening (44) of the device, when the actuation means is completely extended.

In specific embodiments of the invention, the retention means can be a fastening means that is activated by a trigger means, such as a Muscle Wire.

In particular, he separate compartment (30) is inserted linearly into the housing (20), causing the actuation means (50) to be compressed. A tether (82) is attached to the actuation means and extends around the outer surface of the device to be gripped by a fastening retention means (such as a clamping mechanism). The fastening means consists of a protrusion extending through a membrane on the surface of the separate compartment. The protrusion is held in position by a latch and lever mechanism (70C) inside the separate compartment. The catch may be engaged by the temporary action of a magnet or electromagnet. The fastening means allows the tether to be kept under tension, thereby preventing movement of the actuation means. When the sensors (e.g. pH and/or temperature) detect an appropriate change in pH and/or temperature, a signal is sent from the microprocessor to a length of muscle wire, causing the muscle wire to shorten. One end of the muscle wire is attached to the catch and lever mechanism. This shortening disengages the catch and lever mechanism and allows the fastening means to release. The actuation means is then free to eject the separate compartment. As the actuation means travels along the housing, the tether is drawn though the main opening of the device. A closure means (80) is attached at a point on the tether that serves to block the opening (44) of the device when the actuation means is completely extended.

In specific embodiments of the invention, the retention means can be a fastening means that is activated by a trigger means, such as a Shape Memory Alloy (SMA).

In particular, the separate compartment (30) is inserted linearly into the housing (20), causing the actuation means (50) to be compressed. A tether (82) is attached to the actuation means and extends around the outer surface of the device to be gripped by a fastening retention means (such as a clamping mechanism). The fastening means consists of a protrusion extending through a membrane on the surface of the separate compartment. The protrusion is held in position by a latch and lever mechanism inside the separate compartment. The catch may be engaged by the temporary action of a magnet or electromagnet. The fastening means allows the tether to be kept under tension, thereby preventing movement of the actuation means. When the sensors (e.g. pH and/or temperature) detect an appropriate change in pH and/or temperature, a signal is sent from the microprocessor to a length of deformed shape memory alloy, causing the shape memory alloy to revert to its initial form. One end of the shape memory alloy is attached to the catch and lever mechanism. This change in form disengages the catch and lever mechanism and allows the fastening means to release. The actuation means is then free to eject the separate compartment. As the actuation means travels along the housing, the tether is drawn though opening of the device. A closure means (80) is attached at a point on the tether that serves to block the opening (44) of the device, when the actuation means is completely extended.

A second aspect of the invention provides a method of obtaining a sample (e.g. internal substance from the gastrointestinal tract of an animal), comprising the following steps; orally administering the device of the invention to the animal, and recovering the device.

The device can be orally ingested by the animal is recovered from the stool and the sample is easily extracted. The sample is preserved and recovered from the device to perform various biological, chemical and physical tests, such as total nitrogen tests for protein, amino acid analysis using high performance liquid chromatography, measuring the size of the peptides, for example by gel permeation chromatography, assessing immune factors present such as by ELISA, identification, enumeration of microbes using microbiological culture methods or molecular/DNA sequencing or fingerprint analysis or detection of metabolites for example by Mass spectrometry.

In some embodiments, the capsule can be administered to the animal in a fasting state, with food or at an interval before and/or after feeding.

In some embodiment, more than one sampling device can be ingested by the animal. Each device may be ingested by the animal at different times. Each device is capable to take separate samples at different points along the gastrointestinal tract. The sampling devices may be ingested separately and/or are coupled together.

In particular, the sampling device can store and/or obtain a volume of up to about 1 8 to 20 ml depending on the size of the device.

In some embodiments, the method of the invention is carried out on an animal, for example a mammal. The animal may be a human and/or a companion animal, farm animal or production animal, such as a dog, cat, horse, cow, sheep and/or a chicken.

Modifications to the device may be necessary according to the species on which it is used. In particular, smaller devices would be required for smaller animals. Differences in gastrointestinal pH-profile also need to be accounted for, in particular in the case of ruminants.

Methods of detecting the device during its transit along the gastrointestinal tract of an animal are readily known in the art, such as the use of radiography or ultrasound. Other modes of detecting the device, such as inserting a microchip and/or wireless transmission are readily know in the art.

The device can be used in any method where intervention (human or other) is preferably or necessarily avoided. Such methods include taking a sample from processing tanks (for example sewage, food and beverages, manufacture of biologies or chemicals, such as fuels, agricultural systems, for example biofuels or fertiliser and pesticide production, fish talks (household or industrial) hospital or factory pipes. The method involves adding the device to the system described above and ultimately obtaining the device from the system. The device may be retrieved from the same location or from a different location in the system. The device may have travelled through the system before it is retrieved.

The invention will now be further described by way of reference to the following Examples and Figures, which are provided for the purpose of illustration only and are not to be construed as being limiting on the invention. Reference is made to a number of Figures in which:

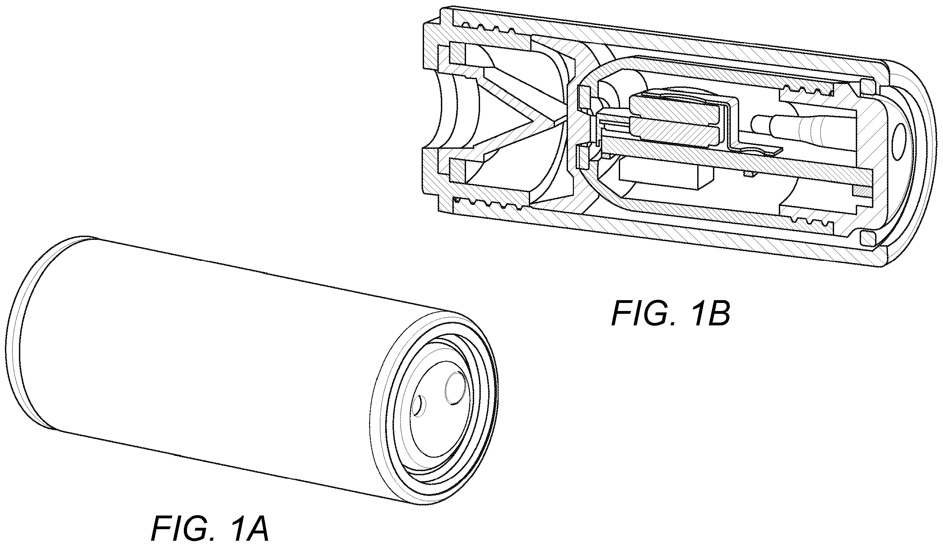

Fig. 1 A and B are views of an example embodiment of the sampling device. Figure 1A shows the external view of a representative device and Figure 1B shows a cross sectional view of the device including the separate electronics component.

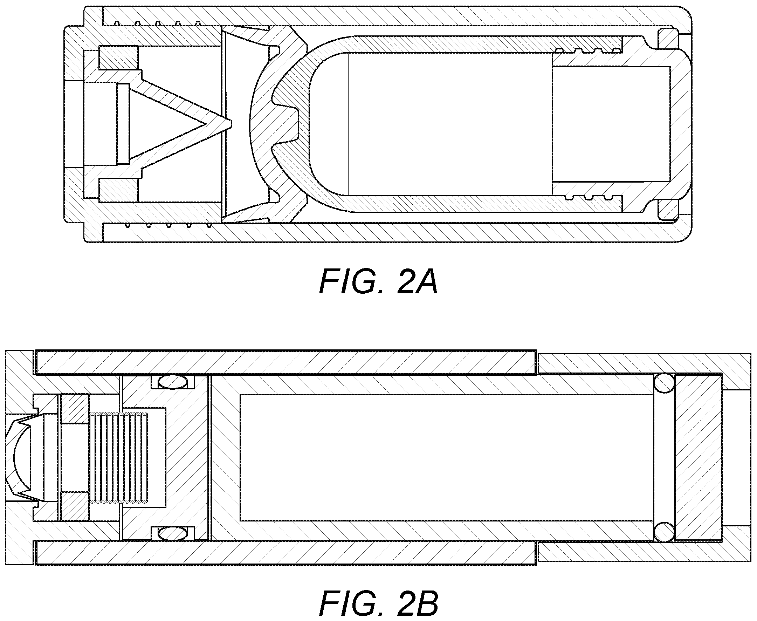

Figs. 2A and 2B is a schematic illustration of the device.

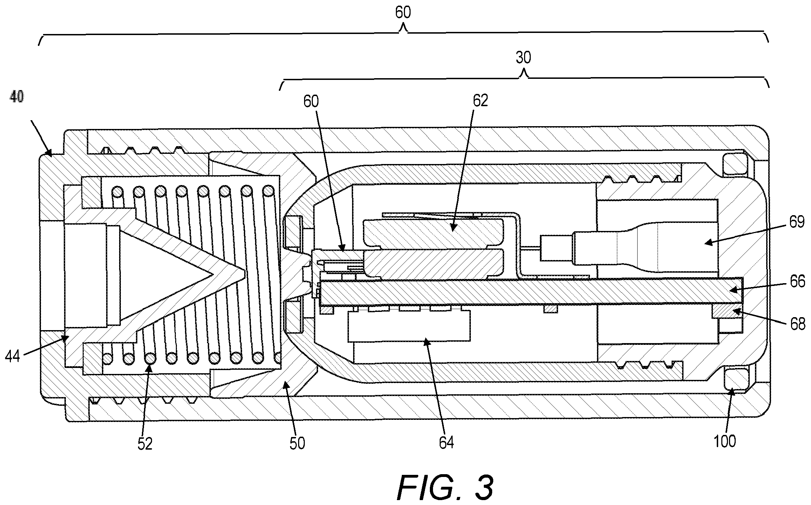

Figure 3 is a schematic view of example of the device.

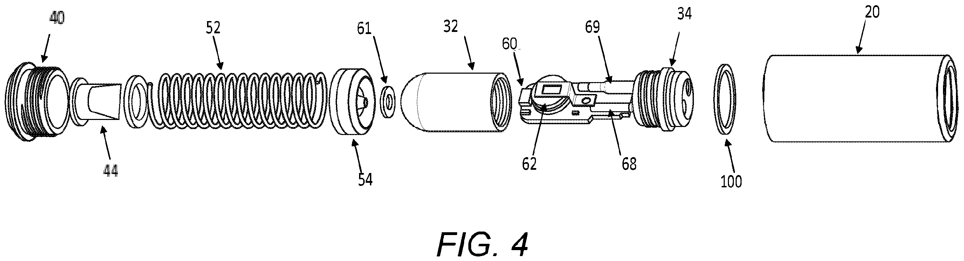

Figure 4 shows an expanded view of the different compartments of one of the embodiments of the device.
Figure 5 shows and illustration of one specific embodiment of the device. Figure 5A shows an illustration of a bayonet mount (100A) and Figure 5B shows an illustration of a specific example of the device, wherein the retention means is a rotary interlocking mechanism (bayonet mount) (100A).
Figure 6 shows and illustration of another specific embodiment of the device. The Figure shows the retention means as a compressed O-ring (100B).
Figure 7 shows and illustration of yet another specific embodiment of the device. The figure illustrates the specific example, wherein the retention means (100) is a fastening means, the retention means is tethered to the closure means (80) that is tethered to the actuation means (50) and a trigger means (70C) is shown.
Figs. 8A to 8L are illustrations of example embodiments of retention means that are interlocking mechanisms.
Figure 8A is a radial interlocking mechanism which is a bowed member friction. Retaining Means - Radial Interlock, bowed member friction. Bowed elements protruding from the sides of the separate compartment interfere with the inner face of the housing to cause friction. This friction prevents the separate compartment from being released. The elements are kept in a bowed configuration by a force generated by the trigger means, for example current being passed through a piezoelectric stack. When the electric current is switched off, the bowed elements relax, reducing the friction and allowing the separate compartment to be released.
Figure 8B is a radial interlocking mechanism which is a circlip. A circlip is fitted into a groove on the outer circumference of the separate compartment. The circlip interferes with a second groove on the inner face of the housing, preventing the separate compartment from being released. The trigger means may be a length of muscle wire that shortens when a current is passed through, squeezing together the circlip such that its circumference is reduced, allowing the separate compartment to be released.
Figure 8C is a rotary interlocking mechanism which is a rotating catch (such as a rotating point catch). A rotatable arm protrudes from the separate compartment to interfere with a lip on the end of the housing, preventing the separate compartment from being released. A trigger means, for example an electronic motor, causes the arm to rotate on a sealed bearing, such that it no longer interferes with the lip, allowing the separate compartment to be released.
Figure 8D is a linear interlocking mechanism which is a deformable barrel. The separate compartment is prevented from being ejected by protrusions on the end of the housing. A section of the housing is made from a deformable material, such as a piezoelectric material. When a current is passed through the piezoelectric material, it deflects outwards away from the separate compartment. The protrusions no longer cause an obstructions and the separate compartment is released.
Figure 8E is a linear interlocking mechanism which is a pin. A pin protrudes though an opening in the separate compartment into a recess hole in the housing, preventing the separate compartment from being released. A trigger means, for example a solenoid device, provides a force which pushes the pin outwards through the hole in the housing, allowing the separate compartment to be released.
Figure 8F is a linear interlocking mechanism which is a bi-metallic latch. A bi-metallic pin protrudes though an opening in the housing and interferes with a recess in the separate compartment preventing the later from being released. A trigger means, for example an electronic heating coil, heats the bi-metallic latch/or pin such that it is deflected or deformed. This change caused the bimetallic latch (or pin) to disengage from the separate compartment, allowing it to be released. (Q=heat)
Figure 8G is a radial interlocking mechanism which is a pull-in catch. Two deformable members protrude from the surface of the separate compartment into holes in the housing, preventing the separate compartment from being ejected. A trigger means, for example a piezoelectric stack, causes the deformable members to be pushed in a direction parallel to the length of the device. This causes the ends of the deformable members to be retracted inwards away from the housing, allowing the separate compartment to be released.
Figure 8H is a linear interlocking mechanism which is a deformable pin. Two elements protrude from the surface of the housing into recesses in the separate compartment, preventing the separate compartment from being released. The elements are deformable, for example being made from a shape memory alloy. When a current is passed through the elements, their consistency changes such that the elements become softer and the force of the actuation means is able to deform them to an extent that the separate compartment is released.
Figure 8I is a rotary interlocking mechanism which are magnets. Magnets embedded in the surface of the separate compartment and the housing are aligned such that there is an attractive force between them. This attractive force prevents the separate compartment from being released. A trigger means, for example an electric motor, causes the magnets to become misaligned, or aligned in such a way that the magnetic poles repel. This removes the attractive force between the magnets, allowing the separate compartment to be released.
Figure 8J is a linear interlocking mechanism which are electromagnets (electromagnetic). The end surface of the separate compartment consists of a ferrous material. The end of the housing is made from a ferrous electromagnet. A current is passed through the electromagnet, generating an attractive force between the end of the housing and the end surface of the separate compartment, preventing the separate compartment from being released. When the current is removed, the attractive force no longer exists and the separate compartment can be released.
Figure 8K is a representation of a retention means that is a thermal sensitive material. The separate compartment is prevented from being released by a material that degrades when heated, such as a wax. Upon heating, the material becomes soft and is no longer able to resist the force of the actuation means and the separate compartment is released.
Figure 8L is a representation of retention means that is a photo sensitive material. The separate compartment is prevented from being released by friction caused by a glue or glue-like material between the separate compartment and the housing. When the material is exposed to light, for example being exposed to a light emitting diode, the (glue) material degrades and the separate compartment is released.
Figure 9 is a plot of time versus pH of the gastrointestinal tract of an animal and shows at which point the device opened and at which point the device captured a sample.
Figure 10 is a schematic diagram showing the different electrical components that can be in the separate component.

The following example details how the device can be used:
The separate compartment (30) is removed from the device and unscrewed to allow access to the onboard electronics. The microprocessor (64) is connected to a serial communications clip to allow configuration of an appropriate sample rate. A new battery (62) is inserted onto the PCB (66) and the separate compartment is then resealed. The electronics record pH data from the point of battery insertion until the battery is removed. Each sampling event is associated with a flash of the LED to indicate to the user that the pill is functioning correctly.

The pH sensor (69) is then calibrated against a known set of pH buffer solutions.

The separate compartment (30) is inserted into the housing (20) along with a washer made of a material that degrades in alkaline pH conditions. This washer acts as a retention means, preventing the separate compartment from being ejected.

The valve assembly is screwed into the housing. This compresses the spring (52) of the actuation means (50), forcing the plunger against the separate compartment. A protrusion on the plunger engages with the switch assembly on the PCB through a membrane on the surface of the separate compartment. This is recorded by the onboard electronics as indicating that the device is in its closed/compressed configuration.

The device is then ready to be administered to the test subject.

During transit along the gastrointestinal tract, the device samples the output of the pH sensor (69) according to the predetermined sampling rate, for example, every 30 seconds. On exit from the stomach, the washer of the retention means begins to degrade in the alkaline environment of the duodenum. When the washer has degraded to such an extent that it is no longer able to withstand the force of the compressed spring (52) of the actuation means (50), the separate compartment (30) is ejected from the device.

As the separate compartment (30) is ejected from the device, the movement of the plunger (54) along the length of the device creates a temporary vacuum that draws a sample of digestive tract contents into the device through an opening (44). During this time, the pressure of the plunger protrusion on the switch assembly (60) is reduced. This is recorded by the onboard electronics as indicating that the device is in its open/uncompressed configuration.

Following transit through the entire digestive tract, the device is recovered on elimination. The device is recovered in two parts. The compartment which contains the sampled digestive tract contents and the separate compartment (30) containing the data relating to the pH recorded during transit and record of the time of sampling represented by the change in the condition of the switch.

The device is unscrewed to retrieve the sampled digestive tract contents. The separate compartment (30) undergoes a second calibration against a known set of pH buffer solutions to check for drift in the output of the pH sensor (69) compared to the initial calibration.

The separate compartment (30) is unscrewed and the battery (62) removed. Data is downloaded from the onboard flash memory through a serial communications clip attached to the microprocessor. This data can be seen in Figure 9.

The device is reprogrammed and ready to use again.

The results in Figure 9 show the pH calibration along the gastrointestinal tract of the animal, at which point the sample was captured and the corresponding pH.

## Claims

1. A sampling device comprising a housing (20), wherein the housing comprises a chamber, at least an opening (44), an actuation means (42) and a separate compartment (30), wherein the separate compartment is releasably retained within the housing by a retention means (100) and wherein the actuation means enables an internal substance to be drawn in through the opening into the chamber and simultaneously pushes the separate compartment along the housing until the separate compartment is ejected from the housing of the sampling device, thereby creating an increased space or volume for receiving and storing a sample within the chamber of the sampling device, **characterised in that** the actuation means is tethered to a closure means (80) and the movement of the actuation means results in the closure means blocking the opening.

2. The sampling device of claim 1, wherein the retention means is a fastening means and/or a material that reacts to changes in pH, temperature, light, solute concentration or an enzyme, optionally wherein the fastening device is tethered to the closure means, optionally wherein the material is degradable, digestible or soluble.

3. The sampling device of claim 2, wherein the fastening means is activated by an interlocking mechanism, optionally wherein the interlocking mechanism is fastened or released by a rotatory, radial or linear motion or means.

4. The sampling device of claim 3, wherein the interlocking mechanism is a catch.

5. The sampling device of claim 2, wherein the fastening means is further activated by a trigger means (70) selected from an electromagnetic or piezoelectric means, a shape memory alloy, muscle wire or a sacrificial fuse.

6. The sampling device of any one of claims 2 to 5, wherein the fastening means is released in response to a release parameter, optionally wherein the fastening means, the interlocking means and/or the trigger means are activated automatically, pre-programmed and/or activated remotely, optionally wherein the release parameter is a change in pH, a change in temperature, a change in moisture, a change in solute concentration, a change in enzyme concentration or a change in light and/or is activated at a pre-determined time and/or at a pre-determined pH and/or at a pre-determined temperature and/or pre-determined moisture level and/or pre-determined solute concentration and/or predetermined enzyme activity or concentration.

7. The sampling device of any one of the previous claims, wherein the actuation means is a plunger (54), optionally wherein the actuation means is a spring (52) and a plunger arrangement, optionally wherein the actuation means is coupled at one end of the housing of the sampling device and the separate compartment is releasably retained by the retention means within the housing at the opposing end of the sampling device, optionally wherein the retention means is activated, the separate compartment is at least partially released and at least partly disengaged from the actuation means.

8. The sampling device of any one of the previous claims, wherein the actuation means is prevented from ejecting from the housing by a stopper means at the end of the housing from which the separate compartment is ejected from the sampling device, optionally wherein the stopper means is a lip, pin, lug or protrusion.

9. The sampling device of any one of the previous claims, wherein the separate compartment comprises of at least a battery (62), a sensor and a microprocessor (64), optionally wherein the sensor is a pH sensor (69) and/or temperature sensor.

10. The sampling device of claim 9, wherein the separate compartment further comprises a pressure switch (60).

11. The sampling device of claim 10, wherein the pressure switch is adapted to record the timing at which the separate compartment is ejected from the housing of the sampling device.

12. The sampling device of any one of the previous claims, wherein the device is reusable.

13. The sampling device of any one of the previous claims, wherein the device is for sampling internal substance from the gastrointestinal tract of an animal.

14. A method of obtaining a sample of the gastrointestinal tract of an animal, comprising the following steps, orally administering the device as claimed in any one of the previous claims to the animal and recovering the device.

15. The method of claim 14, wherein the animal is a human, a companion animal, a farm animal or a production animal such as a dog, cat, horse, cow, sheep and/or chicken.

## Patentansprüche

1. Probenahmevorrichtung, umfassend ein Gehäuse (20), wobei das Gehäuse eine Kammer, mindestens eine Öffnung (44), ein Betätigungsmittel (42) und ein separates Fach (30) umfasst, wobei das separate Fach durch ein Festhaltemittel (100) freigebbar innerhalb des Gehäuses festgehalten wird, und wobei das Betätigungsmittel ermöglicht, dass eine interne Substanz durch die Öffnung hindurch in die Kammer hinein gezogen wird, und gleichzeitig das separate Fach an dem Gehäuse entlang schiebt, bis das separate Fach aus dem Gehäuse der Probenahmevorrichtung ausgeworfen wird, wodurch ein vermehrter Raum oder ein vermehrtes Volumen zum Annehmen und Verwahren einer Probe innerhalb der Kammer der Probenahmevorrichtung erzeugt wird, **dadurch gekennzeichnet, dass** das Betätigungsmittel an einem Verschlussmittel (80) angebracht ist und die Bewegung des Betätigungsmittels dazu führt, dass das Verschlussmittel die Öffnung blockiert.

2. Probenahmevorrichtung nach Anspruch 1, wobei das Festhaltemittel ein Befestigungsmittel und/oder ein Material ist, das auf Änderungen im pH-Wert, in der Temperatur, dem Licht, der Konzentration des gelösten Stoffs oder eines Enzyms reagiert, wobei die Befestigungsvorrichtung gegebenenfalls an dem Verschlussmittel angebracht ist, wobei das Material gegebenenfalls abbaubar, verdaubar oder löslich ist.

3. Probenahmevorrichtung nach Anspruch 2, wobei das Befestigungsmittel durch einen Verriegelungsmechanismus aktiviert wird, wobei der Verriegelungsmechanismus gegebenenfalls durch eine Drehbewegung, radiale oder lineare Bewegung oder ein Drehmittel, radiales oder lineares Mittel befestigt oder freigegeben wird.

4. Probenahmevorrichtung nach Anspruch 3, wobei der Verriegelungsmechanismus eine Raste ist.

5. Probenahmevorrichtung nach Anspruch 2, wobei das Befestigungsmittel des Weiteren durch ein Auslösemittel (70) aktiviert wird, das ausgewählt ist aus einem elektromagnetischen oder piezoelektrischen Mittel, einer Formgedächtnislegierung, Muskeldraht oder einer Opfersicherung.

6. Probenahmevorrichtung nach einem der Ansprüche 2 bis 5, wobei das Befestigungsmittel in Reaktion auf einen Freigabeparameter freigegeben wird, wobei das Befestigungsmittel, das Verriegelungsmittel und/oder das Auslösemittel gegebenenfalls automatisch, vorprogrammiert und/oder fernaktiviert aktiviert werden, wobei der Freigabeparameter gegebenenfalls eine Änderung des pH-Werts, eine Änderung der Temperatur, eine Änderung der Feuchtigkeit, eine Änderung der Konzentration des gelösten Stoffs, eine Änderung der Enzymkonzentration oder eine Änderung des Lichts ist und/oder zu einer vorbestimmten Zeit und/oder bei einem vorbestimmten pH-Wert und/oder bei einer vorbestimmten Temperatur und/oder einem vorbestimmten Feuchtigkeitsniveau und/oder einer vorbestimmten Konzentration eines gelösten Stoffes und/oder vorbestimmter Enzymaktivität oder -konzentration aktiviert wird.

7. Probenahmevorrichtung nach einem der vorhergehenden Ansprüche, wobei das Betätigungsmittel ein Kolben (54) ist, wobei das Betätigungsmittel gegebenenfalls eine Feder (52) und eine Kolbenanordnung ist, wobei das Betätigungsmittel gegebenenfalls an ein Ende des Gehäuses der Probenahmevorrichtung gekoppelt ist und das separate Fach freigebbar durch das Festhaltemittel innerhalb des Gehäuses an dem gegenüber liegenden Ende der Probenahmevorrichtung festgehalten wird, wobei gegebenenfalls, wenn das Festhaltemittel aktiviert wird, das Fach mindestens teilweise freigegeben wird und mindestens teilweise aus dem Eingriff mit dem Betätigungsmittel gelöst wird.

8. Probenahmevorrichtung nach einem der vorhergehenden Ansprüche, wobei durch ein Stoppermittel an dem Ende des Gehäuses, von dem aus das separate Fach aus der Probenahmevorrichtung ausgeworfen wird, verhindert wird, dass das Betätigungsmittel aus dem Gehäuse ausgeworfen wird, wobei das Stoppermittel gegebenenfalls eine Lippe, eine Nadel, eine Lasche oder ein Vorsprung ist.

9. Probenahmevorrichtung nach einem der vorhergehenden Ansprüche, wobei das separate Fach mindestens eine Batterie (62), einen Sensor und einen Mikroprozessor (64) umfasst, wobei der Sensor gegebenenfalls ein pH-Sensor (69) und/oder ein Temperatursensor ist.

10. Probenahmevorrichtung nach Anspruch 9, wobei das separate Fach des Weiteren einen Druckschalter (60) umfasst.

11. Probenahmevorrichtung nach Anspruch 10, wobei der Druckschalter vorgesehen ist, um den Zeitpunkt aufzuzeichnen, an dem das separate Fach aus dem Gehäuse der Probenahmevorrichtung ausgeworfen wird.

12. Probenahmevorrichtung nach einem der vorhergehenden Ansprüche, wobei die Vorrichtung wiederverwendbar ist.

13. Probenahmevorrichtung nach einem der vorhergehenden Ansprüche, wobei die Vorrichtung zur Probenahme von interner Substanz aus dem Gastrointestinaltrakt eines Tieres dient.

14. Verfahren zum Erhalten einer Probe des Gastrointestinaltrakt eines Tieres, umfassend die folgenden Schritte: orales Verabreichen der Vorrichtung gemäß einem der vorhergehenden Ansprüche an das Tier, und Rückgewinnen der Vorrichtung.

15. Verfahren nach Anspruch 14, wobei das Tier ein Mensch, ein Heimtier, ein Nutztier oder ein Produktionstier ist, wie ein Hund, eine Katze, ein Pferd, eine Kuh, ein Schaf und/oder ein Huhn.

## Revendications

1. Dispositif d'échantillonnage comprenant un logement (20), dans lequel le logement comprend une chambre, au moins une ouverture (44), un moyen d'actionnement (42) et un compartiment séparé (30), dans lequel le compartiment séparé est retenu de façon libérable à l'intérieur du logement par un moyen de rétention (100) et dans lequel le moyen d'actionnement permet à une substance interne d'être aspirée, à travers l'ouverture, dans la chambre et pousse simultanément le compartiment séparé le long du logement jusqu'à ce que le compartiment séparé soit éjecté à partir du logement du dispositif d'échantillonnage, ainsi créant un espace ou volume augmenté pour recevoir et stocker un échantillon à l'intérieur de la chambre du dispositif d'échantillonnage, **caractérisé en ce que** le moyen d'actionnement est attaché à un moyen de fermeture (80) et le mouvement du moyen d'actionnement a pour résultat le fait que le moyen de fermeture bloque l'ouverture.

2. Dispositif d'échantillonnage selon la revendication 1, dans lequel le moyen de rétention est un moyen de fixation et/ou un matériau qui réagit à des changements de pH, de température, de lumière, de concentration en soluté ou un enzyme, optionnellement dans lequel le dispositif de fixation est attaché au moyen de fermeture, optionnellement dans lequel le matériau est dégradable, digestible ou soluble.

3. Dispositif d'échantillonnage selon la revendication 2, dans lequel le moyen de fixation est activé par un mécanisme d'interverrouillage, optionnellement dans lequel le mécanisme d'interverrouillage est fixé ou libéré par un mouvement ou moyen rotatif, radial ou linéaire.

4. Dispositif d'échantillonnage selon la revendication 3, dans lequel le mécanisme d'interverrouillage est un encliquetage.

5. Dispositif d'échantillonnage selon la revendication 2, dans lequel le moyen de fixation est en outre activé par un moyen de déclenchement (70) sélectionné parmi un moyen électromagnétique ou piézoélectrique, un alliage à mémoire de forme, un fil musculaire ou un fusible sacrificiel.

6. Dispositif d'échantillonnage selon l'une quelconque des revendications 2 à 5, dans lequel le moyen de fixation est libéré en réponse à un paramètre de libération, optionnellement dans lequel le moyen de fixation, le moyen d'interverrouillage et/ou le moyen de déclenchement sont activés automatiquement, préprogrammés et/ou activés à distance, optionnellement dans lequel le paramètre de libération est un changement de pH, un changement de température, un changement d'humidité, un changement de concentration en soluté, un changement de concentration en enzyme ou un changement de lumière et/ou sont activés à une heure prédéterminée et/ou à un pH prédéterminé et/ou à une température prédéterminée et/ou niveau d'humidité prédéterminé et/ou concentration en soluté prédéterminée et/ou activité d'enzyme ou concentration en enzyme prédéterminée.

7. Dispositif d'échantillonnage selon l'une quelconque des revendications précédentes, dans lequel le moyen d'actionnement est un piston plongeur (54), optionnellement dans lequel le moyen d'actionnement est un ressort (52) et un agencement de piston plongeur, optionnellement dans lequel le moyen d'actionnement est couplé à une extrémité du logement du dispositif d'échantillonnage et le compartiment séparé est retenu de façon libérable par le moyen de rétention à l'intérieur du logement à l'extrémité opposée du dispositif d'échantillonnage, optionnellement dans lequel le moyen de rétention est activé, le compartiment séparé est au moins partiellement libéré et au moins partiellement disjoint du moyen d'actionnement.

8. Dispositif d'échantillonnage selon l'une quelconque des revendications précédentes, dans lequel le moyen d'actionnement est empêché d'effectuer l'éjection, à partir du logement, par un moyen de butée à l'extrémité du logement à partir duquel le compartiment séparé est éjecté à partir du dispositif d'échantillonnage, optionnellement dans lequel le moyen de butée est une lèvre, une goupille, une oreille ou protubérance.

9. Dispositif d'échantillonnage selon l'une quelconque des revendications précédentes, dans lequel le compartiment séparé est composé d'au moins une batterie (62), un capteur et un microprocesseur (64), optionnellement dans lequel le capteur est un capteur pH (69) et/ou capteur de température.

10. Dispositif d'échantillonnage selon la revendication 9, dans lequel le compartiment séparé comprend en outre un pressostat (60).

11. Dispositif d'échantillonnage selon la revendication 10, dans lequel le pressostat est adapté pour enregistrer l'instant auquel le compartiment séparé est éjecté à partir du logement du dispositif d'échantillonnage.

12. Dispositif d'échantillonnage selon l'une quelconque des revendications précédentes, dans lequel le dispositif est réutilisable.

13. Dispositif d'échantillonnage selon l'une quelconque des revendications précédentes, dans lequel le dispositif est destiné à échantillonner une substance interne à partir du tube gastrointestinal d'un animal.

14. Procédé d'obtention d'un échantillon du tube gastrointestinal d'un animal, comprenant les étapes suivantes : l'administration orale du dispositif, selon l'une quelconque des revendications précédente, à l'animal et la récupération du dispositif.

15. Procédé selon la revendication 14, dans lequel l'animal est un humain, un animal de compagnie, un animal de ferme ou un animal de production, tel qu'un chien, un chat, un cheval, une vache, un mouton et/ou une poule.
